# EUROPEAN PATENT SPECIFICATION

(11) **EP 3 706 785 B1**
(45) Date of publication and mention of the grant of the patent: **06.08.2025**
(21) Application number: 18876666.1
(22) Date of filing: 13.11.2018
(51) Int. Cl.: A61K 39/00, A61K 51/10, A61P 35/02, C07K 16/28, A61K 31/555, A61K 31/704, A61K 45/06

(54) **COMBINATION OF RADIONUCLIDE-CONJUGATED ANTIBODIES FOR TREATMENT OF A HEMATOLOGICAL MALIGNANCIES**
KOMBINATIONSTHERAPIE ZUR BEHANDLUNG EINER HÄMATOLOGISCHEN ERKRANKUNG
THÉRAPIE COMBINÉE POUR LE TRAITEMENT D'UNE MALADIE HÉMATOLOGIQUE

(30) Priority: 10.11.2017 US 201762584171 P
(43) Date of publication of application: 16.09.2020
(73) Proprietor: Actinium Pharmaceuticals, Inc., New York, NY 10017 (US)
(72) Inventor: SETH, Sandesh, New York, NY 10128 (US); THOMAS, Keisha, Brooklyn, New York 11226 (US)
(74) Representative: Dentons Patent Solutions Rechtsanwaltsgesellschaft mbH
(86) International application number: PCT/US2018/060737
(87) International publication number: WO 2019/094931

(56) References cited:
- WO-A1-2017/004532
- WO-A1-2018/200841
- US-A1- 2008 267 960
- US-A1- 2011 262 454
- US-A1- 2012 034 185
- US-A1- 2014 219 914
- US-A1- 2016 067 205
- US-A1- 2016 096 892
- US-A1- 2016 101 199
- US-A1- 2017 224 817
- KATHARINA TEILUF ET AL: "[alpha]-Radioimmunotherapy with <sup>213</sup>Bi-anti-CD38 immunoconjugates is effective in a mouse model of human multiple myeloma", ONCOTARGET, vol. 6, no. 7, 10 December 2014 (2014-12-10), pages 4692 - 4703, XP055286741, DOI: 10.18632/oncotarget.2986
- W. F. MAGUIRE ET AL: "Efficient 1-Step Radiolabeling of Monoclonal Antibodies to High Specific Activity with 225Ac for -Particle Radioimmunotherapy of Cancer", THE JOURNAL OF NUCLEAR MEDICINE, vol. 55, no. 9, 30 June 2014 (2014-06-30), US, pages 1492 - 1498, XP055568060, ISSN: 0161-5505, DOI: 10.2967/jnumed.114.138347
- DJORDJE ATANACKOVIC ET AL: "Immunotherapies targeting CD38 in Multiple Myeloma", ONCOIMMUNOLOGY, vol. 5, no. 11, 5 August 2016 (2016-08-05), pages e1217374, XP055505109, DOI: 10.1080/2162402X.2016.1217374
- DANIEL W. SHERBENOU ET AL: "Monoclonal Antibodies in Multiple Myeloma: A New Wave of the Future", CLINICAL LYMPHOMA MYELOMA AND LEUKEMIA, vol. 17, no. 9, 27 June 2017 (2017-06-27), NL, pages 545 - 554, XP055530138, ISSN: 2152-2650, DOI: 10.1016/j.clml.2017.06.030
- GHAI ANCHAL ET AL: "Preclinical Development of CD38-Targeted [ 89 Zr]Zr-DFO-Daratumumab for Imaging Multiple Myeloma", THE JOURNAL OF NUCLEAR MEDICINE, vol. 59, no. 2, 1 February 2018 (2018-02-01), US, pages 216 - 222, XP055810199, ISSN: 0161-5505, Retrieved from the Internet <URL:https://www.ncbi.nlm.nih.gov/pmc/articles/PMC5807532/pdf/216.pdf> DOI: 10.2967/jnumed.117.196063
- JURCIC ET AL.: "Phase I Trial of the Targeted Alpha-Particle Nano-Generator Actinium-225 (225Ac-lintuzumab) (anti- CD 33; HuM195) in Acute Myeloid Leukemia (AML", JOURNAL OF CLINICAL ONCOLOGY, vol. 29, no. 15, 22 September 2016 (2016-09-22), pages 6516, XP055421023, DOI: :10.1200/jco.2011.29.15

## Description

### SEQUENCE LISTING

This application contains a Sequence Listing in computer readable form which is incorporated herein by reference. The sequence listing text file "SequenceListing17039 ST25" submitted via EFS in compliance with 37 CFR §1.52(e)(5) and Rule *13ter .1(a)* is identical to the sequence listing forming part of this international application.

### FIELD OF THE INVENTION

The present invention relates to methods for treating a subject having a proliferative disorder by administration of an anti-CD33 antibody and an anti-CD38 antibody. More specifically, the present invention is related to methods for treating a hematological disease such as relapsed and refractory multiple myeloma by administration of a therapeutic combination of antibodies against CD33 and CD38.

### BACKGROUND OF THE INVENTION

Hematological malignancies, such as multiple myeloma (MM) and acute myeloid leukemia (AML), have historically been treated using high dose chemotherapies and/or radiation. Current treatment protocols generally include a combination of chemotherapeutic agents such as vincristine, carmustine, melphalan, cyclophosphamide, adriamycin, and steroidal agents such as prednisone or dexamethasone. Such treatment, however, yields low remission rates and poor overall survival rates for hematological diseases.

For example, multiple myeloma shows a complete remission rate of only about 5%, and median survival of approximately 36-48 months from the time of diagnosis. Recent advances using high dose chemotherapy followed by autologous bone marrow or peripheral blood mononuclear cell transplantation have increased the complete remission rate and remission duration. Yet overall survival has been only slightly prolonged, and no evidence for a cure has been obtained. Many MM patients will relapse within a few years, and in most cases the relapsed patients do not respond to therapy. For more than 90% of MM patients, the disease becomes chemoresistant.

The commonly applied therapies for AML include chemotherapy and/or radiation, and under certain circumstances, bone marrow transplantation. However, these therapies are relatively toxic to the patient and very often do not lead to a complete cure from the disease. For example, although a complete remission can be achieved for 65-80% of AML patients receiving chemotherapy, most of these patients relapse because the cells that survived the chemotherapy are enriched in AML leukemia stem cells, and constitute a particularly dangerous reservoir of cells capable of re-expanding and causing a relapse.

Phenotypic changes distinguishing cancerous cells from normal cells derived from the same tissue, or cell type, are often associated with one or more changes in the expression of specific gene products, including the loss of normal cell surface components or the gain of others (i.e., antigens not detectable in corresponding normal, non-cancerous cells). The antigens which are expressed by cancerous cells (i.e., in or on the cancerous cells), but not by normal cells, or which are expressed by cancerous cells at levels substantially above those found in normal cells, are often referred to as "tumor-specific antigens" or "tumor-associated antigens." Such tumor-specific antigens may serve as markers of a tumor phenotype, and have been used as targets for cancer immunotherapies.

Cancer immunotherapies have been found to significantly improve the clinical outcomes for patients with a limited subset of cancers. The treatment outcomes for certain hematological diseases, for example, have been improved through the use of targeted immunotherapies. Since most diseases involve several parallel signaling pathways, inhibitors of more than one receptor and/or ligand may provide potential improvement in cancer immunotherapy regimes and clinical treatment outcomes.

Thus, there is a need for improved methods for treating cancers such as hematological cancers using specific combinations of targeted immunotherapies.

### SUMMARY OF THE INVENTION

The present invention is defined by independent claim 1. The dependent claims depict other embodiments of the invention.

The present invention discloses an anti-CD33 antibody and an anti-CD38 antibody for treating a subject having a proliferative disorder comprising an effective amount of the anti-CD33 antibody and the anti-CD38 antibody, wherein the anti-CD33 antibody and the anti-CD38 antibody each independently comprise a label selected from 225Ac; wherein the proliferative disorder is acute myeloid leukemia or multiple myeloma; wherein the anti-CD33 antibody and anti-CD38 antibody are attached to the label by a conjugated chelating agent; wherein the chelating agent attached to the anti-CD38 antibody is 1,4,7,10-tetraazacyclododecance-1,4,7,10-tetraacetic acid (DOTA), and wherein the anti-CD33 antibody is lintuzumab and the anti-CD38 antibody is daratumumab. The methods generally comprise administering an effective amount of an immunotherapy directed to one or more epitopes of CD33 and an effective amount of an immunotherapy directed to one or more epitopes of CD38.

The immunotherapy against the one or more epitopes of CD33 and the one or more epitopes of CD38 may comprise two separate agents, such as an antibody against an epitope of CD33 (anti-CD33 antibody) and an antibody against an epitope of CD38 (anti-CD38 antibody). The anti-CD33 antibody and the anti-CD38 antibody may be administered at the same time, or may be administered sequentially. For example, the anti-CD33 antibody may be administered before the anti-CD38 antibody, after the anti-CD38 antibody, or both before and after the anti-CD38 antibody. Alternatively, the anti-CD38 antibody may be administered before the anti-CD33 antibody, after the anti-CD33 antibody, or both before and after the anti-CD33 antibody.

Alternatively, or in addition, the immunotherapy may comprise one agent, such as a multi-specific antibody against an epitope of CD33 and an epitope of CD38. That is, the immunotherapy may comprise a multi-specific antibody comprising at least a first target recognition component which specifically binds to an epitope of CD33 and a second target recognition component which specifically binds to an epitope of CD38. The antibody may be a humanized monoclonal antibody.

According to certain aspects the anti-CD33 antibody and/or the anti-CD38 antibody may comprise a radioisotope label ("radiolabel"): ¹³¹I, ¹²⁵I, ¹²³I, ⁹⁰Y, ¹⁷⁷Lu, ¹⁸⁶Re, ¹⁸⁸Re, ⁸⁹Sr, ¹⁵³Sm, ³²P, ²²⁵Ac, ²¹³Bi, ²¹³Po, ²¹¹At, ²¹²Bi, ²¹³Bi, ²²³Ra, ²²⁷Th, ¹⁴⁹Tb, ¹³⁷CS, ²¹²Pb and ¹⁰³Pd.

According to certain aspects the anti-CD33 antibody may comprise a humanized antibody against CD33, such as lintuzumab (HuM195), gemtuzumab, or vadastuximab. According to certain aspects of the present invention, the anti-CD38 antibody may comprise a humanized antibody against CD38, such as daratumumab, MOR202, or SAR650984.

According to certain aspects the proliferative disorder may be a hematological disease or disorder. According to certain aspects of the present invention, the hematological disease or disorder may comprise multiple myeloma, acute myeloid leukemia, chronic myeloid leukemia, chronic lymphocytic leukemia, acute lymphoblastic leukemia, non-Hodgkin lymphoma, and Hodgkin lymphoma. According to certain aspects of the present invention, the hematological disease or disorder may comprise relapsed and/or refractory multiple myeloma or acute myeloid leukemia. According to certain aspects of the present invention, the proliferative disorder may be associated with cells expressing CD38, CD33, or both CD33 and CD33.

The uses of the present invention, which include administration of monospecific and/or multi-specific immunological agents, may further comprise administering one or more further therapeutic agents. Such agents may include at least chemotherapeutic agents, anti-inflammatory agents, immunosuppressive agents, immunomodulatory agents, or a combination thereof. Moreover, the one or more further therapeutic agents may comprise an anti-myeloma agent, such as dexamethasone, melphalan, doxorubicin, bortezomib, lenalidomide, prednisone, carmustine, etoposide, cisplatin, vincristine, cyclophosphamide, and thalidomide.

The objects of the present invention will be realized and attained by means of the combinations specifically outlined in the appended claims. The foregoing general description and the following detailed description and examples of this invention are provided to illustrate various aspects of the present invention, and by no means are to be viewed as limiting any of the described embodiments.

### BRIEF DESCRIPTION OF THE DRAWINGS

**FIG. 1** provides the amino acid sequence of human CD33 as shown in GenBank accession number NP_001763.
**FIG. 2** provides the amino acid sequence of human CD38 as shown in GenBank accession number NP_001766.
**FIG. 3** provides a schematic diagram of a method for radiolabeling daratumumab with actinium (²²⁵Ac), wherein panel (**A**) illustrates attachment of the bifunctional chelator S-2-(4-Isothiocyanatobenzyl)-1,4,7,10 tetraazacyclododecanetetraacetic acid (p-SCN-Bn-DOTA; referred to as DOTA in the figures) to the monoclonal antibody daratumumab (DARA); and panel (B) illustrates radiolabeling of the DOTA-DARA conjugate with ²²⁵Ac to provide ²²⁵Ac-DARA.
**FIGS. 4A-4D** provide graphs showing the ability of ²²⁵Ac-DARA conjugates to induce lysis of Daudi cells as compared to unlabeled DARA, ²²⁵Ac-DOTA-IgG conjugates, and untreated cells, wherein **FIGS. 4A-4D** show exposure times of 24, 48, 72, and 96 hours, respectively.
**FIG. 5** provides a summary graph of the data found in **FIGS. 4A-4D**, indicating the percent cell lysis over time for the various concentrations of ²²⁵Ac-DOTA-daratumumab.
**FIGS. 6A** and **6B** provide graphs which indicate the ability of various concentrations of ²²⁵Ac-DOTA-daratumumab conjugates to induce lysis of 28BM and 28PE multiple myeloid cells, respectively, by ADCC over the course of 96 hours.
**FIGS. 7A-7C** provide bar graphs which compare cell lysis results for Daudi, 28BM, and 28PE cells on exposure to various concentration of ²²⁵Ac-DOTA-daratumumab conjugates, wherein **FIG. 7A** shows an exposure time of 48 hours, **FIG. 7B** shows an exposure time of 72 hours, and **FIG. 7C** shows an exposure time of 96 hours.
**FIG. 8** shows biodistribution of ¹¹¹In-DARA in Daudi tumor-bearing SCID mice. Mice were administered a single intraperitoneal (IP) injection of ¹¹¹In-DARA (400 µCi) and imaged with microSPECT/CT at 1, 4, 24 hrs post-injection followed by 2, 3, 7 and 10 days.

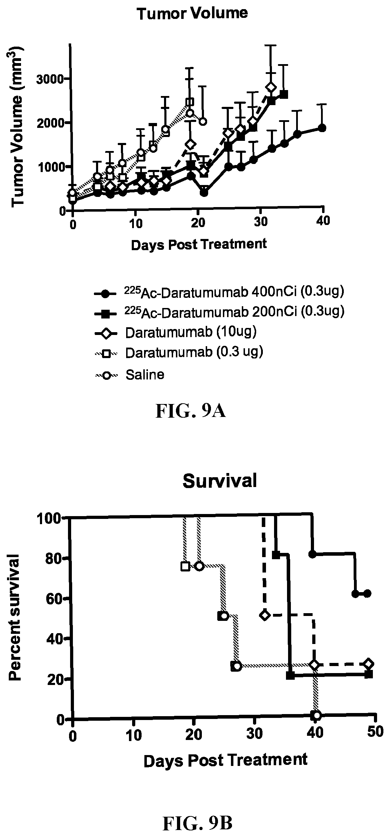

**FIGS 9A-9B** show results from radioimmunotherapy treatment (RIT) of Daudi tumor-bearing SCID mice, wherein **FIG. 9A** shown tumor volume post RIT; and **FIG. 9B** shows a Kaplan-Meier plot of mice survival post RIT. SCID mice were injected with CD38 positive Daudi cells into the right flank. When tumors reached ~200 mm³ mice were treated with a single IP injection of 200 or 400 nCi ²²⁵Ac-DARA (0.3 µg), or an equivalent dose of 0.3 µg naked DARA or a 30-fold higher dose (10 µg) naked DARA, or saline vehicle. Tumor volume was calculated using the formula V=0.5(LW²). Mice were sacrificed when the tumor volume reached 4,000 mm³.

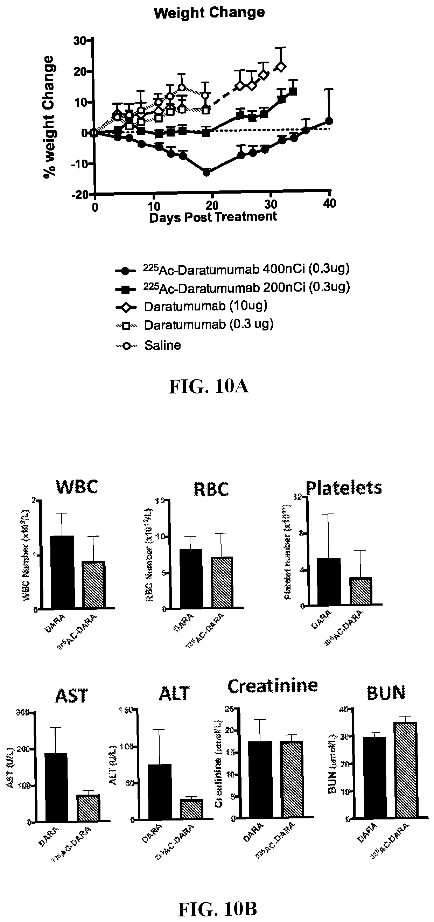

**FIGS. 10A-10B** show results from a safety evaluation of RIT with ²²⁵Ac-DARA, wherein **FIG. 10A** shows weight of the mice post RIT; and **FIG. 10B** shows hematologic, liver and kidney health parameters on day 7 post RIT. SCID mice were injected with CD38 positive Daudi cells into the right flank. When tumors reached ~200 mm³ mice were treated with a single IP injection of 200 or 400 nCi ²²⁵Ac-DARA (0.3 µg), or an equivalent dose of 0.3 µg naked DARA or a 30-fold higher dose (10 µg) naked DARA, or saline vehicle. Mice weight was monitored every 3 days post RIT. Blood was collected 7 days post RIT and analyzed for the indicated parameters.

### DETAILED DESCRIPTION OF THE INVENTION

This invention provides compositions for use for the treatment of hematological diseases and disorders said compositions comprising an anti-CD33 antibody and an anti-CD38 antibody.

### Definitions

In this application, certain terms are used which shall have the meanings set forth as follows.

The singular forms "a," "an," "the" and the like include plural referents unless the context clearly dictates otherwise. Thus, for example, reference to "an" antibody includes both a single antibody and a plurality of different antibodies.

The word "comprising" and forms of the word "comprising", as used in this description and in the claims, does not limit the present invention to exclude any variants or additions. Additionally, although the present invention has been described in terms of "comprising", the processes, materials, and compositions detailed herein may also be described as consisting essentially of or consisting of. For example, while certain aspects of the invention have been described in terms of a method comprising administering a monoclonal antibody against CD33 and CD38 (e.g., two antibodies or a bi-specific antibody), a method "consisting essentially of" or "consisting of" administering the monoclonal antibody against CD33 and CD38 is also within the present scope. In this context, "consisting essentially of" means that any additional components will not materially affect the efficacy of the method.

Moreover, other than in the examples, or where otherwise indicated, all numbers expressing, for example, quantities of ingredients used in the specification are to be understood as being modified in all instances by the term "about". Accordingly, unless indicated to the contrary, the numerical parameters set forth in the following specification are approximations that may vary depending upon the desired properties to be obtained by the present invention. At the very least, and not as an attempt to limit the application of the doctrine of equivalents to the scope of the claims, each numerical parameter should at least be construed in light of the number of reported significant digits and by applying ordinary rounding techniques. Thus, the term "about" when used before a numerical designation, e.g., temperature, time, amount, and concentration, including a range, indicates approximations which may vary by ±10%, ±5%, or ±1%.

As used herein, "administer", with respect to an antibody, means to deliver the antibody to a subject's body via any known method suitable for antibody delivery. Specific modes of administration include, without limitation, intravenous, transdermal, subcutaneous, intraperitoneal, intrathecal and intra-tumoral administration. Exemplary administration methods for antibodies may be as substantially described in International Publication No. WO 2016/187514.

In addition, in this invention, antibodies can be formulated using one or more routinely used pharmaceutically acceptable carriers. Such carriers are well known to those skilled in the art. For example, injectable drug delivery systems include solutions, suspensions, gels, microspheres and polymeric injectables, and can comprise excipients such as solubility-altering agents (e.g., ethanol, propylene glycol and sucrose) and polymers (e.g., polycaprylactones and PLGA's).

As used herein, the term "antibody" includes, without limitation, (a) an immunoglobulin molecule comprising two heavy chains and two light chains and which recognizes an antigen; (b) polyclonal and monoclonal immunoglobulin molecules; (c) monovalent and divalent fragments thereof (e.g., di-Fab), and (d) bi-specific forms thereof. Immunoglobulin molecules may derive from any of the commonly known classes, including but not limited to IgA, secretory IgA, IgG and IgM. IgG subclasses are also well known to those in the art and include, but are not limited to, human IgG1, IgG2, IgG3 and IgG4. Antibodies can be both naturally occurring and non-naturally occurring (e.g., IgG-Fc-silent). Furthermore, antibodies include chimeric antibodies, wholly synthetic antibodies, single chain antibodies, and fragments thereof. Antibodies may be human, humanized or nonhuman.

As used herein, "Immunoreactivity" refers to a measure of the ability of an immunoglobulin to recognize and bind to a specific antigen. "Specific binding" or "specifically binds" or "binds" refers to an antibody binding to an antigen or an epitope within the antigen with greater affinity than for other antigens. Typically, the antibody binds to the antigen or the epitope within the antigen with an equilibrium dissociation constant (KD) of about 1x10⁻⁸ M or less, for example about 1x10⁻⁹ M or less, about 1x10⁻¹⁰ M or less, about 1x10⁻¹¹ M or less, or about 1x10⁻¹² M or less, typically with the KD that is at least one hundred fold less than its KD for binding to a nonspecific antigen (e.g., BSA, casein). The dissociation constant may be measured using standard procedures. Antibodies that specifically bind to the antigen or the epitope within the antigen may, however, have cross-reactivity to other related antigens, for example to the same antigen from other species (homologs), such as human or monkey, for example *Macaca fascicularis* (cynomolgus, cyno), *Pan troglodytes* (chimpanzee, chimp) or *Callithrix jacchus* (common marmoset, marmoset).

As used herein, an "anti-CD33 antibody" is an antibody that binds to any available epitope of CD33. According to certain aspects, the anti-CD33 antibody binds to the epitope recognized by the monoclonal antibody "lintuzumab" or "HuM195." HuM195 is known, as are methods of making it. Moreover, an "anti-CD38 antibody" is an antibody that binds to any available epitope of CD38. According to certain aspects, the anti-CD38 antibody binds to the epitope recognized by the monoclonal antibody "daratumumab." Daratumumab is known, as are methods of making it.

An "epitope" refers to the target molecule site (e.g., at least a portion of an antigen) that is capable of being recognized by, and bound by, an antibody. For a protein antigen, for example, this may refer to the region of the protein (i.e., amino acids, and particularly their side chains) that is bound by the antibody. Overlapping epitopes include at least one to five common amino acid residues. Methods of identifying epitopes of antibodies are known to those skilled in the art and include, for example, those described in Antibodies, A Laboratory Manual, Cold Spring Harbor Laboratory, Ed Harlow and David Lane (1988).

As used herein, "cancer" includes, without limitation, a solid cancer (e.g., a tumor) and a hematologic malignancy.

A "hematologic malignancy", also known as a blood cancer, is a cancer that originates in blood-forming tissue, such as the bone marrow or other cells of the immune system. Hematologic malignancies include, without limitation, leukemias (such as acute myeloid leukemia (AML), acute promyelocytic leukemia, acute lymphoblastic leukemia (ALL), acute mixed lineage leukemia, chronic myeloid leukemia, chronic lymphocytic leukemia (CLL), hairy cell leukemia and large granular lymphocytic leukemia), myelodysplastic syndrome (MDS), myeloproliferative disorders (polycythemia vera, essential thrombocytosis, primary myelofibrosis and chronic myeloid leukemia), lymphomas, multiple myeloma, MGUS and similar disorders, Hodgkin's lymphoma, non-Hodgkin lymphoma (NHL), primary mediastinal large B-cell lymphoma, diffuse large B-cell lymphoma, follicular lymphoma, transformed follicular lymphoma, splenic marginal zone lymphoma, lymphocytic lymphoma, T-cell lymphoma, and other B-cell malignancies.

"Solid cancers" include, without limitation, bone cancer, pancreatic cancer, skin cancer, cancer of the head or neck, cutaneous or intraocular malignant melanoma, uterine cancer, ovarian cancer, prostate cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, pediatric tumors, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally-induced cancers including those induced by asbestos.

According to certain aspects, the antibodies against CD33 and/or CD38 may be labelled with a radioisotope. Methods of labeling these antibodies with radioisotopes, such as ¹³¹I or ²²⁵Ac, are known. These methods are described, for example, in International Publication No. WO 2017/155937.

As used herein, a "radioisotope" can be an alpha-emitting isotope, a beta-emitting isotope, and/or a gamma-emitting isotope. Examples of radioisotopes include the following: ¹³¹I, ¹²⁵I, ¹²³I, ⁹⁰Y, ¹⁷⁷Lu, ¹⁸⁶Re, ¹⁸⁸Re, ⁸⁹Sr, ¹⁵³Sm, ³²P, ²²⁵Ac, ²¹³Bi, ²¹³Po, ²¹¹At, ²¹²Bi, ²¹³Bi, ²²³Ra, ²²⁷Th, ¹⁴⁹Tb, Cs, ²¹²Pb and ¹⁰³Pd. Methods for affixing a radioisotope to an antibody (i.e., "labeling" an antibody with a radioisotope) are well known.

According to certain aspects, when the anti-CD33 or anti-CD38 antibody is radiolabeled with ¹³¹I ("¹³¹I-labeled"), the effective amount is below, for example, 1200 mCi (i.e., where the amount of ¹³¹I administered to the subject delivers a total body radiation dose of below 1200 mCi). According to certain aspects, when the antibody is ¹³¹I-labeled, the effective amount is below 1000 mCi, below 750 mCi, below 500 mCi, below 250 mCi, below 200 mCi, below 150 mCi, below 100 mCi, below 50 mCi, below 40 mCi, below 30 mCi, below 20 mCi or below 10 mCi. According to certain aspects of this method, the effective amount of ¹³¹I-labeled antibody is from 10 mCi to 200 mCi. Examples of effective amounts include, without limitation, from 50 mCi to 100 mCi, from 50 mCi to 150 mCi, from 50 mCi to 200 mCi, from 60 mCi to 140 mCi, from 70 mCi to 130 mCi, from 80 mCi to 120 mCi, from 90 mCi to 110 mCi, from 100 mCi to 150 mCi, 50 mCi, 60 mCi, 70 mCi, 80 mCi, 90 mCi, 100 mCi, 110 mCi, 120 mCi, 130 mCi, 140 mCi, 150 mCi, or 200 mCi. According to certain aspects of this method, the effective amount of ¹³¹I-labeled antibody is from 200 mCi to 1200 mCi. Examples of effective amounts include, without limitation, from 200 mCi to 300 mCi, from 200 mCi to 400 mCi, from 200 mCi to 500 mCi, from 200 mCi to 600 mCi, from 200 mCi to 700 mCi, from 200 mCi to 800 mCi, from 200 mCi to 900 mCi, from 200 mCi to 1000 mCi, from 200 mCi to 1100 mCi, from 300 mCi to 1200 mCi, from 400 mCi to 1200 mCi, from 500 mCi to 1200 mCi, from 600 mCi to 1200 mCi, from 700 mCi to 1200 mCi, from 800 mCi to 1200 mCi, from 900 mCi to 1200 mCi, from 1000 mCi to 1200 mCi, 50 mCi, 100 mCi, 150 mCi, 200 mCi, 300 mCi, 400 mCi, 500 mCi, 600 mCi, 700 mCi, 800 mCi, 900 mCi, 1000 mCi, or 1100 mCi.

According to certain aspects, when the anti-CD33 or anti-CD38 antibody is radiolabeled with ²²⁵Ac ("²²⁵Ac-labeled"), the effective amount is below, for example, 5.0 µCi/kg (i.e., where the amount of ²²⁵Ac administered to the subject delivers a radiation dose of below 5.0 µCi per kilogram of subject's body weight). According to certain aspects, when the antibody is ²²⁵ Ac-labeled, the effective amount is below 4.5 µCi/kg, 4.0 µCi/kg, 3.5 µCi/kg, 3.0 µCi/kg, 2.5 µCi/kg, 2.0 µCi/kg, 1.5 µCi/kg, 1.0 µCi/kg, 0.9 µCi/kg, 0.8 µCi/kg, 0.7 µCi/kg, 0.6 µCi/kg, 0.5 µCi/kg, 0.4 µCi/kg, 0.3 µCi/kg, 0.2 µCi/kg, 0.1 µCi/kg or 0.05 µCi/kg. According to certain aspects, when the antibody is ²²⁵Ac-labeled, the effective amount is from 0.05 µCi/kg to 0.1 µCi/kg, from 0.1 µCi/kg to 0.2 µCi/kg, from 0.2 µCi/kg to 0.3 µCi/kg, from 0.3 µCi/kg to 0.4 µCi/kg, from 0.4 µCi/kg to 0.5 µCi/kg, from 0.5 µCi/kg to 0.6 µCi/kg, from 0.6 µCi/kg to 0.7 µCi/kg, from 0.7 µCi/kg to 0.8 µCi/kg, from 0.8 µCi/kg to 0.9 µCi/kg, from 0.9 µCi/kg to 1.0 µCi/kg, from 1.0 µCi/kg to 1.5 µCi/kg, from 1.5 µCi/kg to 2.0 µCi/kg, from 2.0 µCi/kg to 2.5 µCi/kg, from 2.5 µCi/kg to 3.0 µCi/kg, from 3.0 µCi/kg to 3.5 µCi/kg, from 3.5 µCi/kg to 4.0 µCi/kg, from 4.0 µCi/kg to 4.5 µCi/kg, or from 4.5 µCi/kg to 5.0 µCi/kg. According to certain aspects, when the antibody is ²²⁵Ac-labeled, the effective amount is 0.05 µCi/kg, 0.1 µCi/kg, 0.2 µCi/kg, 0.3 µCi/kg, 0.4 µCi/kg, 0.5 µCi/kg, 0.6 µCi/kg, 0.7 µCi/kg, 0.8 µCi/kg, 0.9 µCi/kg, 1.0 µCi/kg, 1.5 µCi/kg, 2.0 µCi/kg, 2.5 µCi/kg, 3.0 µCi/kg, 3.5 µCi/kg, 4.0 µCi/kg or 4.5 µCi/kg.

According to certain aspects of the present invention, when the antibody is labeled with a radioisotope, the majority of the antibody administered to a subject typically consists of non-labeled antibody, with the minority being the labeled antibody. The ratio of labeled to non-labeled antibody can be adjusted using known methods. Thus, accordingly to certain aspects of the present invention, the anti-CD33 and/or anti-CD38 antibody may be provided in a total protein amount of up to 100mg, such as up to 60 mg, such as 5mg to 45mg, or a total protein amount of between 0.01 mg/kg patient weight to 15.0 mg/kg patient weight, such as between 0.01 mg/kg patient weight to 1.0 mg/kg, or between 0.2 mg/kg patient weight to 0.6 mg/kg patient weight, or 0.3 mg/kg patient weight, or 0.4 mg/kg patient weight, or 0.5 mg/kg patient weight.

According to certain aspects of the present invention, when the anti-CD33 or anti-CD38 antibody is radiolabeled, the radiolabeled anti-CD33 and/or anti-CD38 antibody may comprise a labeled fraction and an unlabeled fraction, wherein the ratio of labeled : unlabeled may be from about 0.01:10 to 1:1, such as 0.1:10 to 1:1 labeled : unlabeled. Moreover, the radiolabeled anti-CD33 and/or anti-CD38 antibody may be provided as a single dose composition tailored to a specific patient, wherein the amount of labeled and unlabeled anti-CD33 and/or anti-CD38 in the composition may depend on at least a patient weight, age, and/or disease state or health status, such as detailed in International Publication No. WO 2016/187514.

As used herein, the term "subject" includes, without limitation, a mammal such as a human, a non-human primate, a dog, a cat, a horse, a sheep, a goat, a cow, a rabbit, a pig, a rat and a mouse. Where the subject is human, the subject can be of any age. For example, the subject can be 60 years or older, 65 or older, 70 or older, 75 or older, 80 or older, 85 or older, or 90 or older. Alternatively, the subject can be 50 years or younger, 45 or younger, 40 or younger, 35 or younger, 30 or younger, 25 or younger, or 20 or younger. For a human subject afflicted with cancer, the subject can be newly diagnosed, or relapsed and/or refractory, or in remission.

As used herein, "treating" a subject afflicted with a cancer shall include, without limitation, (i) slowing, stopping or reversing the cancer's progression, (ii) slowing, stopping or reversing the progression of the cancer's symptoms, (iii) reducing the likelihood of the cancer's recurrence, and/or (iv) reducing the likelihood that the cancer's symptoms will recur. According to certain preferred aspects, treating a subject afflicted with a cancer means (i) reversing the cancer's progression, ideally to the point of eliminating the cancer, and/or (ii) reversing the progression of the cancer's symptoms, ideally to the point of eliminating the symptoms, and/or (iii) reducing or eliminating the likelihood of relapse (i.e., consolidation, which ideally results in the destruction of any remaining cancer cells).

"Chemotherapeutic", in the context of this invention, shall mean a chemical compound which inhibits or kills growing cells and which can be used or is approved for use in the treatment of cancer. Exemplary chemotherapeutic agents include cytostatic agents which prevent, disturb, disrupt or delay cell division at the level of nuclear division or cell plasma division. Such agents may stabilize microtubules, such as taxanes, in particular docetaxel or paclitaxel, and epothilones, in particular epothilone A, B, C, D, E, and F, or may destabilize microtubules such as vinca alcaloids, in particular vinblastine, vincristine, vindesine, vinflunine, and vinorelbine.

"Synergistic combinations," as used herein, are combinations of monotherapies that may provide a therapeutic effect that is comparable to the effectiveness of a monotherapy, while reducing adverse side effects, e.g. damage to non-targeted tissues, immune status, and other clinical indicia. Alternatively, synergistic combinations may provide for an improved effectiveness, which may be measured by total tumor cell number; length of time to relapse, and other indicia of patient health.

Synergistic combinations of the present invention combine an agent that is targeted to inhibit or block CD38 function, and an agent that is targeted to inhibit or block CD33 function. The combination may be provided with a combination of agents, e.g. two distinct monoclonal antibodies, or may be provided as a multi-specific agent, e.g. bi-specific antibody.

"Therapeutically effective amount" or "effective amount" refers to an amount effective, at dosages and for periods of time necessary, to achieve a desired therapeutic result. A therapeutically effective amount may vary according to factors such as the disease state, age, sex, and weight of the individual, and the ability of a therapeutic or a combination of therapeutics to elicit a desired response in the individual. Exemplary indicators of an effective therapeutic or combination of therapeutics include, for example, improved well-being of the patient, reduction in a tumor burden, arrested or slowed growth of a tumor, and/or absence of metastasis of cancer cells to other locations in the body.

"Inhibits growth" refers to a measurable decrease or delay in the growth of a malignant cell or tissue (e.g., tumor) in vitro or in vivo when contacted with a therapeutic or a combination of therapeutics or drugs, when compared to the decrease or delay in the growth of the same cells or tissue in the absence of the therapeutic or the combination of therapeutic drugs. Inhibition of growth of a malignant cell or tissue in vitro or in vivo may be at least about 10%, 20%, 30%, 40%, 50%, 60%, 70%, 80%, 90%, 95%, 96%, 97%, 98%, 99%, or 100%.

Throughout this application, various publications are cited to describe more fully the state of the art to which this invention pertains.

Unless otherwise defined, all technical and scientific terms used herein have the same meaning as commonly understood by one of ordinary skill in the art to which the present invention belongs. Although methods and materials similar or equivalent to those described herein can be used in the practice or testing described herein, suitable methods and materials are described below.

### Aspects of the Invention

The present invention is related to methods for treating a proliferative disease by administering an effective amount of an antibody against an epitope of CD33 and an effective amount of an antibody against an epitope of CD38. The antibodies may be administered sequentially or they may be administered simultaneously. Moreover, each antibody may be administered according to a specific dosing schedule, wherein the method provides for administration of each antibody according to the dosing schedule sequentially (one antibody dosing schedule is completed before the next antibody dosing schedule is started) or simultaneously.

The methods disclosed herein may be used to treat a proliferative disorder such as a hematological disease or disorder. The hematological disease or disorder may comprise multiple myeloma (MM), acute myeloid leukemia (AML), chronic myeloid leukemia (CIVIL), chronic lymphocytic leukemia (CLL), acute lymphoblastic leukemia (ALL), non-Hodgkin lymphoma (NHL), and Hodgkin lymphoma (HL). According to certain aspects of the present invention, the hematological disease or disorder may comprise relapsed and/or refractory multiple myeloma or acute myeloid leukemia.

The methods disclosed herein may be used to inhibit growth and/or proliferation of a cell expressing CD33 and/or CD38. The methods disclosed herein may also be used to treat a disease or disorder involving cells expressing or overexpressing CD33 and/or CD38.

Leukemia stem cells, which have been particularly well characterized for AML, express a characteristic set of cell-surface antigens including, among others, CD33. The CD33 antigen is expressed on the blast cells of most cases of AML; about 85-90% of AML cases express the CD33 antigen. Moreover, the CD33 antigen is expressed on virtually all cases of CML. Patients older than 60 years have a poor prognosis with only 10% to 15% of 4-year disease-free survival for AML. This high relapse rate for AML patients and the poor prognosis for older patients highlight the urgent need for novel therapeutics preferentially targeting CD33⁺ cells.

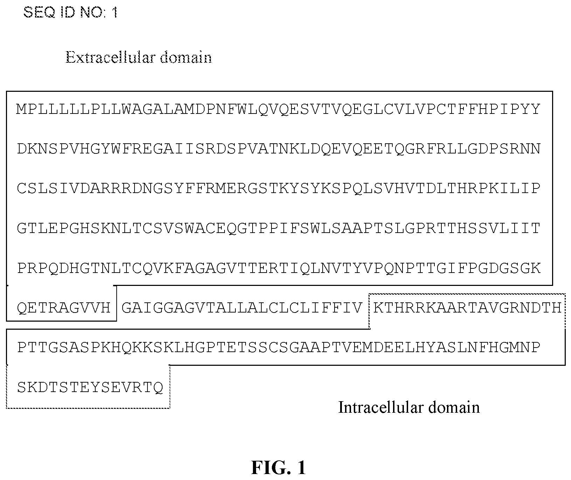

CD33 is a type I transmembrane receptor glycoprotein that may function as a sialic acid-dependent cell adhesion molecule. Expressed on early myeloid progenitor and myeloid leukemic (e.g., AML) cells, CD33 is not expressed on stem cells. Human CD33 has an amino acid sequence shown in GenBank accession number NP_001763 and in SEQ ID NO: 1 (FIG. 1). CD33 is a 67 kDa single pass transmembrane protein with amino acid residues 1-259 representing the extracellular domain, amino acids 260-282 representing the helical transmembrane domain, and amino acids 283-364 representing the cytosolic domain (intracellular). There are at least three known single nucleotide polymorphisms ("SNPs") in the extracellular domain of CD33 (i.e., W22R, R69G, S128N). Therefore, the extracellular domain of *Homo sapiens* CD33 can have the amino acid sequence of SEQ ID NO: 1 with any one or more of these SNPs.

The CD33 protein has structural similarity to immunoreceptors, and its normal function is thought to serve as a negative regulator of immunoreceptor (ITAM) signal transduction. Recent studies suggest a role for CD33 in the modulation of inflammatory and immune responses through a dampening effect on tyrosine kinase-driven signaling pathways. For example, in vitro studies have demonstrated that CD33 constitutively suppresses the production of pro-inflammatory cytokines such as IL-1β, TNF-α, and IL-8 by human monocytes in a sialic acid ligand-dependent and SOCS3-dependent manner. Conversely, reduction of cell surface CD33 or interruption of sialic acid binding can increase p38 mitogen-activated protein kinase (MAPK) activity and enhance cytokine secretion as well as cytokine-induced cellular proliferation.

Antibodies against CD33, such as lintuzumab (HuM195), gemtuzumab, and vadastuximab have been, and are currently being evaluated in the clinic for their efficacy to treat hematological malignancies and plasma cell disorders, including acute myeloid leukemia (AML). Each antibody has been found to bind to a different portion of the extracellular region of CD33, and each demonstrates different clinical responses (e.g., anti-tumor effects). Gemtuzumab is available from Pfizer as Mylotarg^{™}, and vadastuximab is available from Seattle Genetics as Vadastuximab talirine.

For example, the antibody lintuzumab (HuM195) has demonstrated anti-leukemic effects in treatment of AML. HuM195 is a recombinant humanized anti-CD33 monoclonal antibody originally produced by Protein Design Labs, Inc. (Fremont, California). M195 is a monoclonal IgG2a antibody that binds CD33. M195 is derived from a mouse immunized with live human leukemic myeloblasts. HuM195 was constructed by grafting complementarity-determining regions of M195 into a human IgG1 framework and backbone. HuM195 induced antibody-dependent cell-mediated cytotoxicity using human peripheral blood mononuclear cells as effectors. Four clinical trials have investigated native (i.e., unconjugated) HuM195 alone in patients with relapsed or refractory AML and CML. Fever, chills, and nausea were the most common toxicities. Human anti-human antibody responses were not seen. Beneficial biologic activity in terms of reduction in marrow blast cells was seen in some patients. Those who benefited the most had fewer blasts at the beginning of therapy, suggesting that HuM195 may be more effective in the treatment of minimal residual or cytoreduced disease.

Multiple myeloma cells uniformly overexpress CD38, which is a 45 kD type II transmembrane glycoprotein with a long C-terminal extracellular domain and a short N-terminal cytoplasmic domain. The CD38 protein is a bi-functional ectoenzyme that can catalyze the conversion of NAD⁺ into cyclic ADP-ribose (cADPR), and cADPR into ADP-ribose, and thus regulates extracellular NAD⁺ concentrations. Moreover, cADPR has been shown to be a second messenger for intracellular Ca²⁺ mobilization. Thus, CD38 may be an essential component for regulation of intracellular Ca²⁺ flux.

CD38 expression is also upregulated in a variety of other malignant hematological diseases, including but not limited to B-cell chronic lymphocytic leukemia, B-cell acute lymphocytic leukemia, Waldenström macroglobulinemia, primary systemic amyloidosis, mantlecell lymphoma, pro-lymphocytic/myelocytic leukemia, acute myeloid leukemia, chronic myeloid leukemia, follicular lymphoma, NK-cell leukemia and plasma-cell leukemia.

Moreover, expression of CD38 has been described on epithelial/endothelial cells of different origin, including glandular epithelium in prostate, islet cells in pancreas, ductal epithelium in glands, including parotid gland, bronchial epithelial cells, cells in testis and ovary, and tumor epithelium in colorectal adenocarcinoma. Thus, diseases where CD38 expression could be involved include, but are not limited to, broncho-epithelial carcinomas of the lung, breast cancer evolving from malignant proliferation of the epithelial lining in ducts and lobules of the breast, pancreatic tumors evolving from the b-cells (e.g., insulinomas), and tumors evolving from epithelium in the gut (e.g. adenocarcinoma and squamous cell carcinoma).

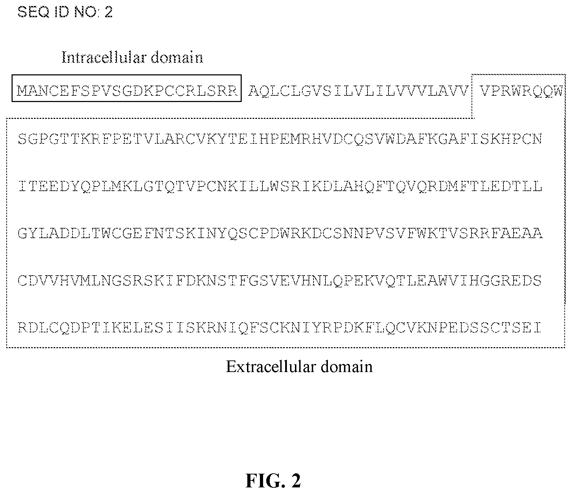

Human CD38 has an amino acid sequence shown in GenBank accession number NP_001766 and in SEQ ID NO: 2 (**FIG. 2**). CD38 is a single pass type II membrane protein with amino acid residues 1-21 representing the cytosolic domain, amino acid residues 22-42 representing the transmembrane domain, and residues 43-300 representing the extracellular domain of CD38.

Antibodies against CD38, such as daratumumab, MOR202, or SAR650984, have been, and are currently being evaluated in the clinic for their efficacy to treat hematological malignancies and plasma cell disorders, including multiple myeloma. Each antibody has been found to bind to a different portion of the extracellular region of CD38, and each demonstrates different clinical responses (*e.g*., anti-tumor effects).

Daratumumab, available from Johnson & Johnson (Janssen Biotech)/Genmab as Darzalex^{®} is described in the publication to de Weers et. al., "Daratumumab, a Novel Therapeutic Human CD38 Monoclonal Antibody, Induces Killing of Multiple Myeloma and Other Hematological Tumors," J Immunology, 2010, 186(3): 1840-1848. MOR202, available from Celgene Corp./Morphosys, is described in U.S. Patent No. 8,877,899. SAR650984, available from Sanofi/Immunogen as Isatuximab, is described in the publication to Park et al., "SAR650984: A Potent Anti-CD38 Therapeutic Antibody with Three Mechanisms of Action (Apoptosis, ADCC, CDC) for Hematological :Malignancies," BLOOD, vol. 112, No. 11, Nov. 2008, p 951, and Deckert et el., "SAR650984, A Novel Humanized CD38-Targeting Antibody, Demonstrates Potent Antitumor Activity in Models of Multiple Myeloma and Other CD38+ Hematologic Malignancies," Clin Cancer Res 2014; 20:4574-83, and U.S. Patent No. 8,153,765. **Table** 1 below lists a number of antibodies, or fragments thereof, that bind CD38, and their epitope (binding site) on the CD38 molecule, some or all of which may be useful according to various aspects of the present invention.

**TABLE 1**

| **Name** | **Type** | **CD38 Epitope** (amino acids) | Reference |
|---|---|---|---|
| SUN4B7 | IgG1 | 254-275 | 1 |
| OKT10 | IgG1 | 280-298 | 1 |
| HB7 | IgG1 | 254-275 | 1, 2 |
| IB4 | IgG2a | 220-241 and 273-285 | 1 |
| IB6 | IgG2b | Not determined | 1 |
| AT1 | IgG1 | 254-275 | 1 |
| SAR650984 or 38SB19 | IgG1 (human) | 107-120, 125, 146, 155, 189, 193, 194 and 226 | 3 |
| Daratumumab | IgG1 (human) | 233-246 and 267-280 | 4 |
| MOR202 | IgG1 (human) | Not determined | 5 |

| | | | |
|---|---|---|---|
| 1) Tissue Antigens 2000, (56):539-547 and BMC Immunology 2004, (5):21 2) J. Biol. Chem. 2011, (286):22170-22177 3) U.S. Pat. No. 8,153,765 and Clin Cancer Res 2014, 20(17):4574-83 4) J Immunol 2011, (186):1840-1848 5) U.S. Pat. No. 8,877,899 | | | |

Proposed methods by which these antibodies eliminate CD33- and CD38-positive cells include antibody-dependent cellular cytotoxicity (ADCC), complement-dependent cytotoxicity (CDC), and apoptosis.

"Antibody-dependent cellular cytotoxicity", "antibody-dependent cell-mediated cytotoxicity" or "ADCC" is a mechanism for inducing cell death that depends upon the interaction of antibody-coated target cells with effector cells possessing lytic activity, such as natural killer (NK) cells, monocytes, macrophages and neutrophils via Fc gamma receptors (FcyR) expressed on effector cells. For example, NK cells express FcγRIIIa, whereas monocytes express FcyRI, FcγRII and FcvRIIIa. Death of the antibody-coated target cell, such as CD38-expressing cells, occurs as a result of effector cell activity through the secretion of membrane pore-forming proteins and proteases.

"Complement-dependent cytotoxicity", or "CDC", refers to a mechanism for inducing cell death in which an Fc effector domain of a target-bound antibody binds and activates complement component C1q, which in turn activates the complement cascade leading to target cell death. Activation of complement may also result in deposition of complement components on the target cell surface that facilitate ADCC by binding complement receptors (e.g., CR3) on leukocytes.

"Apoptosis" refers to a mechanism of programmed cell death wherein antibody binding to the target cell disrupts integral cell signaling pathways and results in cell selfdestruction.

To assess ADCC activity of an antibody that binds to a specific antigen, such as an antibody against CD38 or CD33, the antibody may be added to antigen-expressing cells in combination with immune effector cells, which may be activated by the antigen-antibody complexes resulting in cytolysis of the antigen-expressing cells, respectively. Cytolysis is generally detected by the release of a label (e.g. radioactive substrates, fluorescent dyes or natural intracellular proteins) from the lysed cells. Exemplary effector cells for such assays include peripheral blood mononuclear cells (PBMC) and NK cells.

As example, in an exemplary assay for ADCC activity of an anti-CD38 antibody, CD38-expressing cells may be labeled with ⁵¹Cr and washed extensively. The anti-CD38 antibodies may be added to the CD38-expressing cells at various concentrations, and the assay started by adding effector cells (NK cells from peripheral blood mononuclear cells, for example). After incubation for various time intervals at 37°C, assays are stopped by centrifugation and ⁵¹Cr release from lysed cells is measured in a scintillation counter. The percentage of cellular cytotoxicity may be calculated as the percent maximal lysis which may be induced by adding 3% perchloric acid to the CD38-expressing cells.

In an exemplary assay for cytotoxicity, tetrazolium salt may be added to the CD38-expressing cells treated with various amounts of the anti-CD38 antibody. In living mitochondria, the XTT is reduced to an orange product by mitochondrial dehydrogenase and transferred to the cell surface. The orange product can be optically quantified and reflects the number of living cells. Alternatively, esterases from living cells are known to hydrolyze the colorless calcenin into as fluorescent molecule. The fluorescence can be measured and quantified, and reflects the number of living cells in the sample. The total amount of dead cells may be measured using propidium iodide, which is excluded from live cells by intact membranes. The fluorescence due to the propidium iodide in dead cells may be quantified by flow-cytometry.

In order to assess CDC, complement protein may need to be included in an assay for cytotoxicity. Measurement of apoptosis induction does not require addition of NK cells or complement protein in an assay for cytotoxicity.

Treatment of certain cancers with monoclonal antibodies against CD33 has met with varied success. In initial studies with an unconjugated murine anti-CD33 antibody (M195), a few patients had transient decreases in peripheral blast counts at a saturating or supra-saturating dose. Subsequent studies employed a humanized version of M195, lintuzumab (HuM195), which had greater than 8-fold higher binding avidity than M195 and, unlike M195, demonstrated antibody-dependent cell-mediated cytotoxicity (ADCC). Still, while limited studies pointed toward some activity in acute promyelocytic leukemias (APL) when used in in patients with minimal residual disease, lintuzumab has very modest activity as a single agent in AML even at supra-saturating doses that fully blocked CD33 binding sites throughout a 4-week period, with the infrequent achievement of complete or partial remissions limited to patients with low tumor burden. Efficacy can perhaps be increased if supra-saturating doses are given repeatedly, as suggested by a small trial in which very high doses of lintuzumab were given weekly for 5 weeks and then every other week for patients with clinical benefit.

On the other hand, treatment of various hematological malignancies with unlabeled (*i.e.,* "naked") monoclonal antibodies against CD38 found success in recent clinical trials. One such antibody, daratumumab, is currently marketed as a treatment for multiple myeloma under the name Darzalex^{®} by Janssen Biotech, Inc. The treatment regime includes an initial 8 week dosing schedule of 1 dose weekly by infusion of 16mg/kg patient weight. Pretreatment with antipyretics and antihistamines are meant to ameliorate some of the more common and serious side effects of infusion. For example, infusion reactions that occur in greater than half of all patients include bronchospasms, hypoxia, dyspnea, hypertension, laryngeal edema, and pulmonary edema, and can occur up to 48 hours after treatment. Side effects that occur in greater than 20 percent of treated patients include at least fatigue, nausea, diarrhea, constipation, muscle spasms, back pains, chills, insomnia, cough, and dyspnea. Thus, while treatment with a naked antibody against CD38 has found some success for the treatment of a hematological malignancy, the side effects of the treatment are fairly serious and may be related to the high protein dose of the monoclonal antibody.

Disclose herein are methods of treating a proliferative disease or disorder which includes administration of antibodies against both CD33 and CD38. Such a combination may deliver synergistic effects, such as by providing a therapeutic effect comparable to the effectiveness of a monotherapy while reducing adverse side effects of the monotherapies, and/or an improved therapeutic effectiveness measurable by a reduction in the total tumor cell number, increase in the length of time to relapse, and/or other indicia of patient health.

The combination may be provided with a combination of agents, e.g. an antibody against CD33 and an antibody against CD38, or may be provided as a multi-specific agent, e.g. multi-specific antibody against a first target recognition component and a second target recognition component (i.e., an epitope of CD33 and an epitope of CD38, respectively).

According to certain aspects of the present invention, either or both of the anti-CD33 and anti-CD38 antibodies may be labeled with a radiolabel. When the immunotherapy includes a multi-specific antibody, either one or both of the first target recognition component and the second target recognition component may comprise a radiolabel.

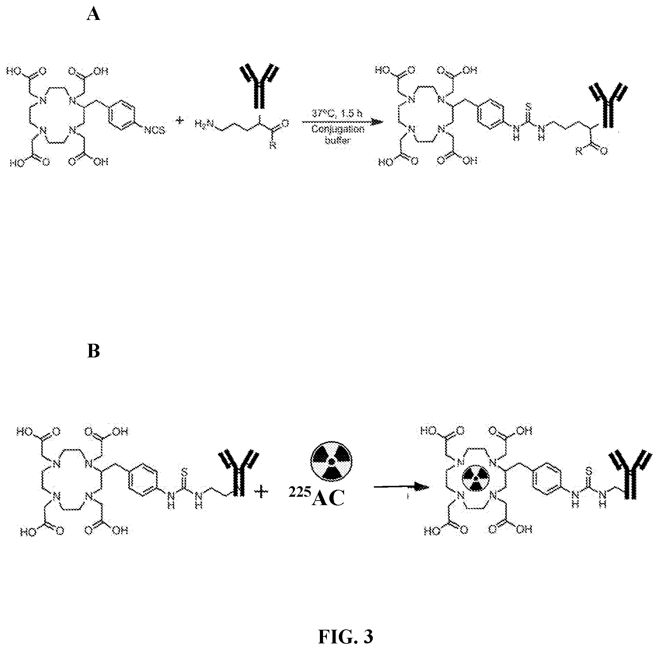

The antibodies may be labelled with the radionuclide by any means known in the art. According to one aspect of the invention, the radionuclide may be attached or chelated by a chelating agent which is conjugated to the monoclonal antibody such as substantially described in co-pending provisional Patent Application 62/539,114. That is, the radionuclide labelled monoclonal antibody may be prepared by first forming a chelator conjugated antibody ("conjugated antibody"), and then chelating a radionuclide with the conjugated antibody to form the radiolabeled antibody, as shown in FIG. 3. A radionuclide may be chelated by the conjugated antibody at any time following conjugation.

According to certain aspects of the present invention, the radiolabeled monoclonal antibody may be relatively stable. For example, greater than 75% of the monoclonal antibody labelled with ²²⁵Ac- or ¹³¹I may remain intact after storage for 24 hours at 4°C.

According to certain aspects of the present invention, the radiolabeled monoclonal antibody may exhibit essentially the same immunoreactivity to the antigen as a control monoclonal antibody, wherein the control monoclonal antibody comprises an un-labeled monoclonal antibody against the same epitope of the antigen (i.e., CD33 or CD38) as the ²²⁵Ac- or ¹³¹I-labelled monoclonal antibody.

According to the present invention, the radiolabeled anti-CD33 antibody is radiolabeled HuM195. Radiolabeled antibodies envisioned include, without limitation, ¹³¹I-HuM195, ¹²⁵I-HuM195, ¹²³I-HuM195, ⁹⁰Y-HuM195, ¹⁷⁷Lu-HuM195, ¹⁸⁶Re-HuM195, ¹⁸⁸Re-HuM195, ⁸⁹Sr-HuM195, ¹⁵³Sm-HuM195, ³²P-HuM195, ²²⁵Ac-HuM195, ²¹³Bi- HuM195, ²¹³Po-HuM195, ²¹¹At-HuM195, ²¹²Bi-HuM195, ²¹³Bi-HuM195, ²²³Ra-HuM195, ²²⁷Th-HuM195, ¹⁴⁹Tb-HuM195, ¹³¹I-HuM195, ¹³⁷Cs-HuM195, ²¹²Pb-HuM195 and ¹⁰³Pd-HuM195. Preferably, the radiolabeled BC8 is ¹³¹I-HuM195 or ²²⁵Ac-HuM195.

According to the present invention, the radiolabeled anti-CD38 antibody is radiolabeled daratumumab (Dara). Radiolabeled antibodies envisioned include, without limitation, ¹³¹I-Dara, ¹²⁵I-Dara, ¹²³I-Dara, ⁹⁰Y-Dara, ¹⁷⁷Lu-Dara, ¹⁸⁶Re-Dara, ¹⁸⁸Re-Dara, ⁸⁹Sr-Dara, ¹⁵³Sm-Dara, ³²P-Dara, ²²⁵Ac-Dara, ²¹³Bi-Dara, ²¹³Po-Dara, ²¹¹At-Dara, ²¹²Bi-Dara, ²¹³Bi-Dara, ²²³Ra-Dara, ²²⁷Th-Dara, ¹⁴⁹Tb-Dara, ¹³¹I-Dara, ¹³⁷Cs-Dara, ²¹²Pb-Dara and ¹⁰³Pd-Dara. Preferably, the radiolabeled BC8 is ¹³¹I-Dara or ²²⁵Ac-Dara.

According to certain aspects the effective amount of ¹³¹I-HuM195 and/or ¹³¹I-Dara is from 10 mCi to 200 mCi. Examples of effective amounts include, without limitation, from 50 mCi to 100 mCi, from 50 mCi to 150 mCi, from 50 mCi to 200 mCi, from 60 mCi to 140 mCi, from 70 mCi to 130 mCi, from 80 mCi to 120 mCi, from 90 mCi to 110 mCi, from 100 mCi to 150 mCi, 50 mCi, 60 mCi, 70 mCi, 80 mCi, 90 mCi, 100 mCi, 110 mCi, 120 mCi, 130 mCi, 140 mCi, 150 mCi, or 200 mCi. According to certain aspects of this method, the effective amount of ¹³¹I-HuM195 and/or ¹³¹I-Dara is from 200 mCi to 1200 mCi. Examples of effective amounts include, without limitation, from 200 mCi to 300 mCi, from 200 mCi to 400 mCi, from 200 mCi to 500 mCi, from 200 mCi to 600 mCi, from 200 mCi to 700 mCi, from 200 mCi to 800 mCi, from 200 mCi to 900 mCi, from 200 mCi to 1000 mCi, from 200 mCi to 1100 mCi, from 300 mCi to 1200 mCi, from 400 mCi to 1200 mCi, from 500 mCi to 1200 mCi, from 600 mCi to 1200 mCi, from 700 mCi to 1200 mCi, from 800 mCi to 1200 mCi, from 900 mCi to 1200 mCi, from 1000 mCi to 1200 mCi, 50 mCi, 100 mCi, 150 mCi, 200 mCi, 300 mCi, 400 mCi, 500 mCi, 600 mCi, 700 mCi, 800 mCi, 900 mCi, 1000 mCi, or 1100 mCi.

According to certain aspects of the present invention, the effective amount of ²²⁵Ac-HuM195 and/or ²²⁵Ac-Dara is below, for example, 5.0 µCi/kg (i.e., where the amount of ²²⁵Ac-labeled antibody administered to the subject delivers a radiation dose of below 5.0 µCi per kilogram of subject's body weight). According to certain aspects, the effective amount of ²²⁵Ac-HuM195 and/or ²²⁵Ac-Dara is below 4.5 µCi/kg, 4.0 µCi/kg, 3.5 µCi/kg, 3.0 µCi/kg, 2.5 µCi/kg, 2.0 µCi/kg, 1.5 µCi/kg, 1.0 µCi/kg, 0.9 µCi/kg, 0.8 µCi/kg, 0.7 µCi/kg, 0.6 µCi/kg, 0.5 µCi/kg, 0.4 µCi/kg, 0.3 µCi/kg, 0.2 µCi/kg, 0.1 µCi/kg or 0.05 µCi/kg. According to certain aspects, the effective amount of ²²⁵Ac-HuM195 and/or ²²⁵Ac-Dara is from 0.05 µCi/kg to 0.1 µCi/kg, from 0.1 µCi/kg to 0.2 µCi/kg, from 0.2 µCi/kg to 0.3 µCi/kg, from 0.3 µCi/kg to 0.4 µCi/kg, from 0.4 µCi/kg to 0.5 µCi/kg, from 0.5 µCi/kg to 0.6 µCi/kg, from 0.6 µCi/kg to 0.7 µCi/kg, from 0.7 µCi/kg to 0.8 µCi/kg, from 0.8 µCi/kg to 0.9 µCi/kg, from 0.9 µCi/kg to 1.0 µCi/kg, from 1.0 µCi/kg to 1.5 µCi/kg, from 1.5 µCi/kg to 2.0 µCi/kg, from 2.0 µCi/kg to 2.5 µCi/kg, from 2.5 µCi/kg to 3.0 µCi/kg, from 3.0 µCi/kg to 3.5 µCi/kg, from 3.5 µCi/kg to 4.0 µCi/kg, from 4.0 µCi/kg to 4.5 µCi/kg, or from 4.5 µCi/kg to 5.0 µCi/kg. According to certain aspects, the effective amount of ²²⁵Ac-HuM195 and/or ²²⁵Ac-Dara is 0.05 µCi/kg, 0.1 µCi/kg, 0.2 µCi/kg, 0.3 µCi/kg, 0.4 µCi/kg, 0.5 µCi/kg, 0.6 µCi/kg, 0.7 µCi/kg, 0.8 µCi/kg, 0.9 µCi/kg, 1.0 µCi/kg, 1.5 µCi/kg, 2.0 µCi/kg, 2.5 µCi/kg, 3.0 µCi/kg, 3.5 µCi/kg, 4.0 µCi/kg or 4.5 µCi/kg.

According to certain aspects of the present invention, the ²²⁵Ac-labeled anti-CD33 and/or anti-CD-38 antibodies may be administered as one or more doses according to any of the dosing schedules listed herein until the subject has received a cumulative radiation dose of 1 µCi/kg, 2 µCi/kg, 3 µCi/kg, 4 µCi/kg, 5 µCi/kg, 6 µCi/kg, 7 µCi/kg, 8 µCi/kg, 9 µCi/kg, or 10 µCi/kg of patient weight.

According to certain aspects of the present invention, an effective amount of the anti-CD33 antibody may comprises a dose of 0.5 to 4 uCi/kg of subject's body weight, and an effective amount of the anti-CD38 antibody comprises a dose of 0.5 to 4 uCi/kg of subject's body weight. In general, the effective amount of the anti-CD33 antibody comprises a dose of less than 16mg/kg body weight, such as from 0.01mg/kg to 5mg/kg, and the effective amount of the anti-CD38 antibody comprises a dose of less than 16mg/kg body weight, such as from 0.01mg/kg to 5mg/kg.

According to certain aspects of the present invention, the effective amount of the anti-CD33 antibody is a maximum tolerated dose (MTD) of the anti-CD33 antibody, and the effective amount of the anti-CD38 antibody is a maximum tolerated dose (MTD) of the anti-CD38 antibody.

According to certain aspects of the present invention, the anti-CD33 antibody and the anti-CD38 antibody may be administered at the same time. As such, they may be provided in a single composition, or they may be mixed into a single composition just prior to administration. Alternatively, the anti-CD33 antibody and the anti-CD38 antibody may be administered sequentially. As such, the anti-CD33 antibody may be administered before the anti-CD38 antibody, after the anti-CD38 antibody, or both before and after the anti-CD38 antibody. Moreover, the anti-CD38 antibody may be administered before the anti-CD33 antibody, after the anti-CD33 antibody, or both before and after the anti-CD33 antibody.

According to certain aspects of the present invention, the anti-CD33 antibody may be administered according to a dosing schedule selected from the group consisting of once every week, once every two weeks, once every three weeks, once every four weeks, once every five weeks, once every six weeks, once every seven weeks, and once every eight weeks throughout a treatment period, wherein the treatment period includes at least two doses. As such, the anti-CD38 antibody may be administered within 7 days of administration of the anti-CD33 antibody for at least a portion of the treatment period of the anti-CD33 antibody.

According to certain aspects of the present invention, the anti-CD38 antibody may be administered according to a dosing schedule selected from the group consisting of once every week, once every two weeks, once every three weeks, once every four weeks, once every five weeks, once every six weeks, once every seven weeks, and once every eight weeks throughout a treatment period, wherein the treatment period includes at least two doses. As such, the anti-CD33 antibody may be administered within 7 days of administration of the anti-CD38 antibody for at least a portion of the treatment period of the anti-CD38 antibody.

According to certain aspects of the methods of the present invention, the anti-CD33 antibody is administered according to an anti-CD33 dosing schedule and the anti-CD38 antibody is administered according to an anti-CD38 dosing schedule, wherein the anti-CD33 dosing schedule and the anti-CD38 dosing schedule each comprise two or more doses.

According to certain aspects of the present invention, the anti-CD33 antibody may be administered via two injections separated by 7, 14, 21, 28, 35, 42, 49, 56, and 63 days. According to certain aspects of the methods of the present invention, the anti-CD38 antibody may be administered via two injections separated by 7, 14, 21, 28, 35, 42, 49, 56, and 63 days.

According to certain aspects of the present invention, the anti-CD33 antibody may be administered via two injections or infusions separated by 1, 2, 3, 4, 5, 6, or 7 days. According to certain aspects of the methods of the present invention, the anti-CD38 antibody may be administered via two injections or infusions separated by 1, 2, 3, 4, 5, 6, or 7 days.

According to certain aspects of the present invention, each injection or infusion may have a duration of less than 1 day, such as less than 12 hours, 10 hours, 8 hours, or even less than 6 hours. According to certain aspects of the present invention, each injection or infusion may have a duration of less 180 minutes, such as less than 120 minutes, or even less than 60 minutes. The injection or infusion duration may depend on the antibody, and/or whether the antibody is radiolabeled or remains un-labelled, and/or the radiation dose delivered by a radiolabeled antibody. For example, longer injection times may be used for radiolabeled anti-CD33 and anti-CD38 antibodies than for un-labelled anti-CD33 and anti-CD38 antibodies.

The uses of the present invention, which include administration of monospecific and/or multi-specific immunological agents, may further comprise administering one or more further therapeutic agents. The additional therapeutic agents may be relevant for the disease or condition to be treated. Such administration may be simultaneous, separate or sequential with the administration of the effective amount of each antibody agent detailed herein. For simultaneous administration, the agents may be administered as one composition (antibody and therapeutic agent) or as separate compositions, as appropriate.

Exemplary additional therapeutic agents include at least chemotherapeutic agents, anti-inflammatory agents, immunosuppressive agents, immunomodulatory agents, cytokines, hormones, or a combination thereof. Exemplary cytokines include at least granulocyte colony-stimulating factor. Moreover, the one or more further therapeutic agents may comprise an antimyeloma agent, such as dexamethasone, melphalan, doxorubicin, bortezomib, lenalidomide, prednisone, carmustine, etoposide, cisplatin, vincristine, cyclophosphamide, and thalidomide.

According to certain aspects of the present invention, the methods may further comprise administration of a cytokine such as granulocyte colony-stimulating factor (GCSF) after administration of one or both of the anti-CD33 and anti-CD38 antibodies. The GCSF may be administered, for example, 7, 8, 9, 10, or 11 days after administration of one or both of the anti-CD33 and anti-CD38 antibodies.

The therapeutic agents may be administered according to any standard dose regime known in the field. For example, therapeutic agents may be administered at concentrations in the range of 1 to 500 mg/m², the amounts being calculated as a function of patient surface area (m²).

Maximal lysis via CDC and CDCC using Darzalex^{®}, an un-labeled anti-CD38 therapeutic currently on the market, has been reported in the literature to occur at 1ug/ml and 0.10ug/ml, respectively. As indicated above, the ²²⁵Ac- or ¹³¹I-labelled monoclonal antibodies against CD33 and/or CD38 may be at least 5-fold more effective at causing cell death than the unlabeled antibodies. Without wishing to be bound by one theory, such an increase in the cytotoxicity of the radiolabeled anti-CD33 and/or anti-CD38 may include cell death via additional mechanisms, such as, for example, apoptosis.

Disclosed herein are methods for treating a subject having a hematological malignancy, for inhibiting growth and/or proliferation of a cell expressing CD33 and/or CD38, and for treating a disease or disorder involving cells expressing CD33 and/or CD38.

According to certain aspects of the present invention, the hematological malignancy may be multiple myeloma. According to certain aspects of the invention the cell expressing CD33 and/or CD38 may be a multiple myeloma cell. According to certain aspects of the invention, the disease or disorder may be relapsed or refractory multiple myeloma.

According to certain aspects the at least one dose of the dosage regimen comprises an amount of the ²²⁵Ac- or ¹³¹I-labelled monoclonal antibody against CD33 and/or CD38 that is 5-fold lower, or 10-fold lower, or 20-fold lower, or 100-fold lower, than a dose in a dosage regime comprising only a control monoclonal antibody (un-labeled CD33 and/or un-labeled CD38). The control monoclonal antibody generally comprises an un-labeled monoclonal antibody against the same epitope of CD33 and/or CD38, respectively, as the ²²⁵Ac- or ¹³¹I-labelled monoclonal antibody.

According to certain aspects the dosage regime comprises at least one less dose than a control dosage regime, wherein the control dosage regime includes administration, either alone or in combination with one or more additional therapeutic agents, of an un-labeled monoclonal antibody against the same epitope of CD38 as the ²²⁵Ac- or ¹³¹I-labelled monoclonal antibody. According to certain aspects of the present invention, the dosage regime comprises at least two fewer doses than a control dosage regime, wherein the control dosage regime includes administration, either alone or in combination with one or more additional therapeutic agents, of an un-labeled monoclonal antibody against the same epitope of CD38 as the ²²⁵Ac- or ¹³¹I-labelled monoclonal antibody.

According to certain aspects of the present invention, the dosage regime may comprise 10% fewer doses than the control dosage regime, such as 20% fewer doses, or 30% fewer doses, or 40% fewer doses, or 50% fewer doses, or 60% fewer doses, or 70% fewer doses, or 80% fewer doses, or even 90% fewer doses, than the control dosage regime.

According to certain aspects of the present invention, the control monoclonal antibody may comprise daratumumab administered at a dose of about 16 mg/kg patient weight. According to certain aspects of the present invention, the control dosage regime may include 8 doses, administered at one dose per week for at least 8 weeks.

### EXAMPLES

### EXAMPLE 1: CD33 specificity and stability

Lintuzumab conjugated with Actinium-225 (Ac²²⁵) was tested for cytotoxicity against specific cell types which express CD33. For example, suspensions of HL60 (leukemia cells) were incubated with various doses of radiolabeled lintuzumab (lintuzumab- Ac²²⁵), and the dose at which 50% of the cells were killed (LD₅₀) was found to be 8 pCi per mL of cell suspension.

In studies to access the reactivity of the radiolabeled lintuzumab with peripheral blood and bone marrow cells from nonhuman primate and human frozen tissues, the radiolabeled lintuzumab showed reactivity with mononuclear cells only, demonstrating specificity. Moreover, in studies to determine the stability of the radiolabel on the antibody, 10 normal mice (8 week old Balb/c female mice from Taconic, Germantown, New York) were injected in the tail with 300 nCi radiolabeled lintuzumab (in 0.12ml). Serum samples taken over a 5 day period showed that the ²²⁵Ac remained bound to the lintuzumab, demonstrating the stability of the radiolabel on the antibody *in vivo.*

A maximum tolerated dose (MTD) of a single injection of the Ac²²⁵-labeled lintuzumab was determined to be 3uCi/kg patient weight. As a split dose (e.g., 2 equal doses administered 4-7 days apart), the MTD was determined to be 2uCi/kg per dose, or 4uCi/kg total. This data was determined by injections into patients with relapsed/refractory AML: 21 patients were injected with increasing doses of the radiolabeled lintuzumab - 0.5uCi/kg to 4uCi/kg. Determination of MTD was based on the severity of the adverse effects observed at each dose level. Anti-leukemic effects included elimination of peripheral blood blasts in 13 of 19 evaluable patients. Twelve of 18 patients who were evaluable at 4 weeks following treatment had reductions in bone marrow blasts, including nine with reductions ≥ 50%. Three patients treated with 1uCi/kg, 3uCi/kg and 4uCi/kg respectively had ≤ 5% blasts after therapy.

### EXAMPLE 2: CD33 human maximal tolerated dose and efficacy

A Phase I trial will be used to determine the MTD of fractionated doses of lintuzumab-Ac²²⁵ followed by Granulocyte Colony Stimulating factor (GCSF) support in each cycle. A cycle in general is approximately 42 days. A cycle starts with administration of a fractionated dose of Lintuzumab-Ac²²⁵ on Day 1 followed by the administration of GCSF on Day 9 and continuing GCSF per appropriate dosing instructions until absolute neutrophil count (ANC) is greater than 1,000, which is expected to occur within 5 - 10 days. On Days 14, 21, 28, 35 and 42 peripheral blood will be assessed for paraprotein burden. A bone marrow aspirate will be performed to assess plasmocyte infiltration on Day 42. If a response is a partial response or better but less than a complete response on Day 42, and the patient remains otherwise eligible, the patient will be re-dosed in a new cycle at the same dose level no sooner than 60 days after Day 1 of the first cycle. In absence of dose limiting toxicities, cycles will continue using the above described algorithm until the patient has received a cumulative dose of 4 µCi/kg of lintuzumab-Ac²²⁵.

### EXAMPLE 3: Labeling of CD38 with Actinium (Ac²²⁵)

Daratumumab is an immunoglobulin G1 kappa (IgG1κ) cytolytic human monoclonal antibody directed against the CD38 antigen and is approved to treat patients with multiple myeloma. The dosing schedule is rigorous, requiring infusions weekly (up to week 8), bi-monthly (up to week 24), and monthly (post week 25) until disease progression. Daratumumab induces cell lysis by both antibody dependent cellular cytotoxicity (ADCC) and complement dependent cytotoxicity (CDC) mechanisms. The efficient targeting of the CD38 antigen by daratumumab positions it as an attractive vehicle to direct the ²²⁵Ac warhead to its target cells, which may increase the efficacy of daratumumab in multiple myeloma.

Methods: Daratumumab was labeled with ²²⁵Ac and the stability and immunogenicity of the resulting construct, daratumumab-²²⁵Ac was characterized. The ability of the naked daratumumab, daratumumab-²²⁵Ac, and irrelevant IgG-²²⁵Ac to induce cell lysis in multiple myeloma cell lines with a range of CD38 antigen expression levels was assessed at various time points and concentrations.

Results: Importantly, immunogenicity was preserved upon labeling daratumumab with the chelator and ²²⁵Ac. Concentrations of antibodies and ²²⁵Ac-labeled constructs ranging from 0.01 µg/mL to 0.06 µg/mL were tested in each of the four cell lines. The ratio of activity : antibody utilized was 10 nCi:0.01 µg/mL. Treatment of Daudi cells with 0.01 µg/mL and 0.06 µg/mL of daratumumabZ-²²⁵Ac resulted in 25% and 95% of cell death at 72 h, respectively, versus 5-6% cell death in the daratumumab only group. A similar time- and concentration- dependent cell death was demonstrated in the patient derived cell lines, 28PE and 28BM. No time or concentration dependent killing was observed when CD38-positive cell lines were treated with the similar activities of radiolabeled isotype-matching human control antibody IgG-²²⁵Ac, or when the negative CD38 expressing multiple myeloma cell line, U266 was treated with daratumumab-²²⁵Ac.

Conclusion: We have demonstrated the ability to label a CD38 targeting antibody with ²²⁵Ac. The high potency, precision and short range of the alpha emitter, ²²⁵Ac improves the efficacy of daratumumab more than 10-fold, essentially utilizing the antibody as a vehicle while still preserving the immune functions of daratumumab. ²²⁵Ac is an effective payload due to its high linear energy transfer properties and short path length in tissues. The consequences of a more effective cell lysis agent on dosing concentration and frequency are being evaluated as further development of this promising therapeutic approach to treatment of CD38 positive cancers is warranted.

### EXAMPLE 4: Degree of Chelation of Actinium (Ac²²⁵) by daratumumab and immunoreactivity of Actinium (Ac²²⁵) labeled daratumumab

A monoclonal antibody against CD38, daratumumab, was labelled with ²²⁵Ac according to methods disclosed herein. The degree of chelation of ²²⁵Ac by an immunoconjugate of the chelator 1,4,7,10-tetraazacyclododecane-1,4,7,10-tetraacetic acid (DOTA) and the monoclonal antibody daratumumab was measured and found to be at least 80 percent for conjugation reactions performed at 37°C or room temperature, and at various molar excesses (5M to 50M) of the DOTA chelator to the daratumumab. A

DOTA-daratumumab at 0.3 mg/ml in 0.15M ammonium acetate buffer, pH 6.5 was labelled with ²²⁵Ac (chelation of ²²⁵Ac by DOTA) in 0.01M HCL at 1uCi/ug daratumumab for 60 minutes at 37°C or room temperature. Stability of the radiolabeled anti-CD38 was measured on storage at two different temperatures - 4°C and 37°C. At both temperatures, greater than 75% of the ²²⁵Ac-labelled monoclonal antibody remains intact after storage for 24 hours at 4 °C, and greater than 60% of the ²²⁵Ac-labelled monoclonal antibody remains intact after storage for 24 hours at room temperature.

Immunoreactivity of the radiolabeled anti-CD38 was also tested. Binding of ²²⁵Ac-DOTA-daratumumab conjugates to various concentrations of CD38 is essentially unchanged when compared to an un-labeled anti-CD38, even for ²²⁵Ac-DOTA-daratumumab conjugates from conjugation reactions between DOTA and daratumumab performed at 37°C or room temperature, and at various molar excesses of DOTA to daratumumab.

### EXAMPLE 5: Cytotoxocity of Actinium (Ac²²⁵) labeled daratumumab

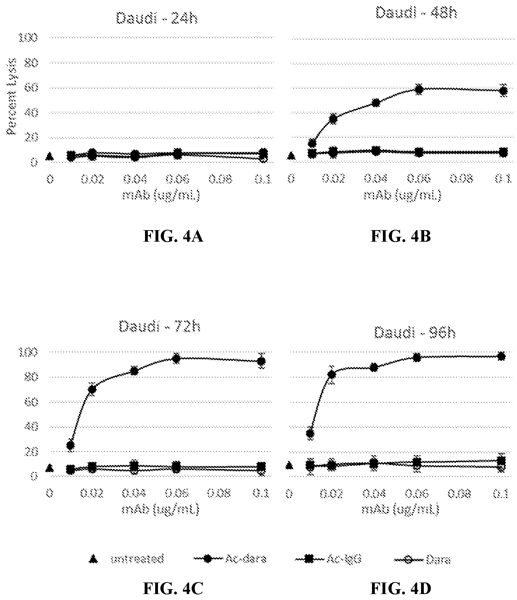

The cytotoxicity of the ²²⁵Ac-DOTA-daratumumab conjugates was then measured against various B-cell types. For example, shown in **FIGS. 4A-4D** are results from an XTT assay for treatment of Daudi cells with ²²⁵Ac-DOTA-daratumumab conjugates. The XTT assay uses XTT tetrazolium to measure the percentage of intact cells. It can be seen that lysis of Daudi cells by ²²⁵Ac-DOTA-IgG conjugates approaches 95% to 100%, while the control daratumumab shows little to no effect at the low concentrations tested in the assay (i.e., same cytotoxicity as the control cell). **FIGS. 4A-4D** show exposure times of 24, 48, 72, and 96 hours, respectively.

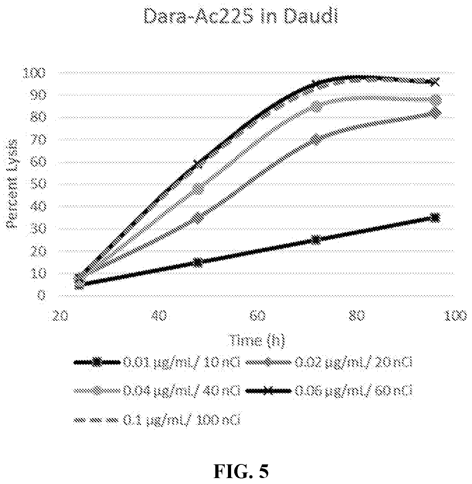

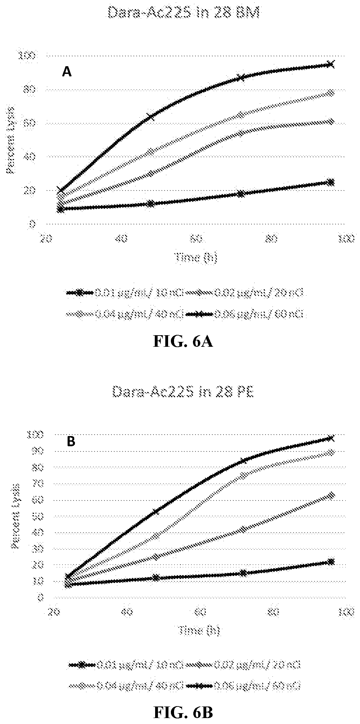

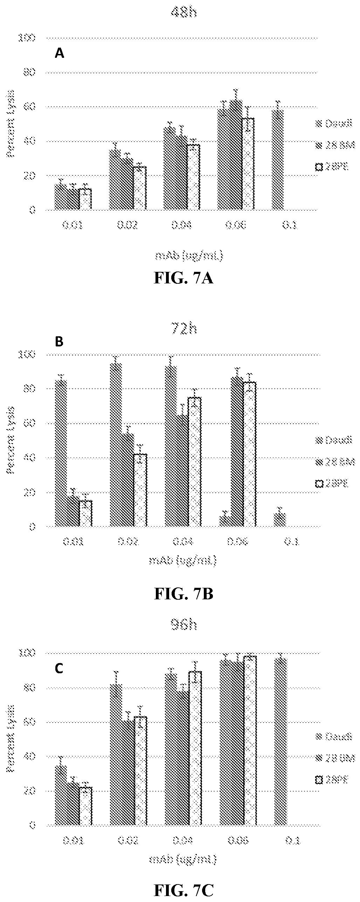

**FIG. 5** provides a summary graph of the data found in **FIG. 4A-4D**, indicating the percent cell lysis over time for the various concentrations of ²²⁵Ac-DOTA-daratumumab. **FIG. 6A-6B** provides graphs which indicate the ability of various concentrations of ²²⁵Ac-DOTA-daratumumab conjugates to induce lysis of 28BM and 28PE multiple myeloid cells, respectively, by ADCC over the course of 96 hours. **FIG. 7A-7C** provides bar graphs which compare cell lysis results for Daudi, 28BM, and 28PE cells on exposure to various concentration of ²²⁵Ac-DOTA-daratumumab conjugates, wherein panels A-C show exposure times of 48, 72, and 96 hours, respectively.

### EXAMPLE 6: Animal models

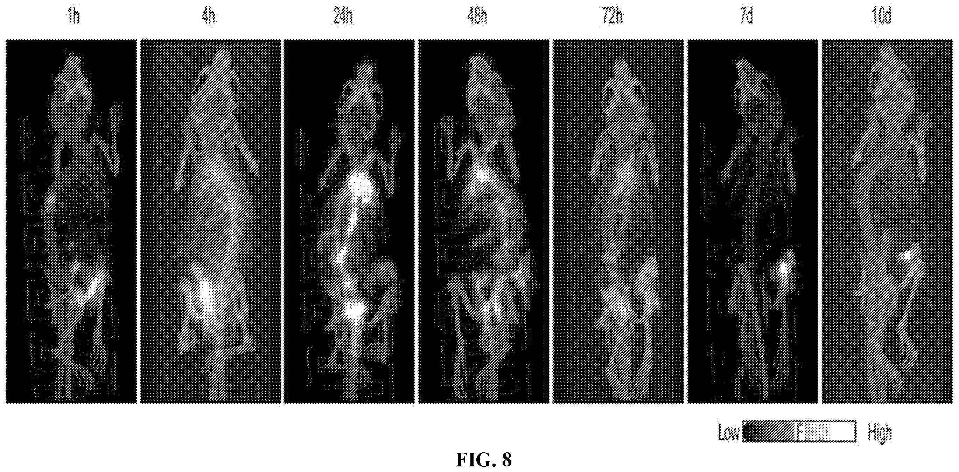

We performed microSPECT/CT imaging of ¹¹¹In-daratumumab (¹¹¹In-DARA) in Daudi tumor-bearing mice to ascertain its specific uptake in the tumors (**FIG. 8**). For tumor induction, female SCID mice (CB17/Icr-Prkdc^{scid}/IcrIcoCrl, procured from Charles River Laboratories) were injected subcutaneously with 5x10⁶ Daudi cells in 50 µL of saline into the right flank. Tumor growth was measured with electronic calipers every 3 days, and tumor volume was calculated using the formula V=0.5(LxW²), with width being the shorter tumor diameter. On day 14 post tumor cell inoculation, when tumors reached an average volume of ~200 mm³, mice were either imaged by microSPECT/CT or treated with ¹¹¹In-DARA. For imaging, 3 mice were injected intraperitoneally (IP) with 400 µCi ¹¹¹In-DARA and imaged on a microSPECT/CT (MI Labs, Netherlands) at 1, 4, 24 hrs post-injection followed by 2, 3, 7 and 10 days.

MicroSPECT/CT imaging demonstrated noticeable localization of ¹¹¹In-DARA in tumors at 24 hrs post administration. ¹¹¹In-DARA continued to concentrate in the tumor up to 10 days, while activity had cleared from the rest of the body. **FIG. 8** shows representative images from one mouse over the entire time course.

To evaluate the side effects of the radioimmunotherapy (RIT), the tumor-bearing mice were randomized into the groups of 5 animals and injected IP with either: 100 µL saline, or unlabeled DARA at concentrations of 10 or 0.3 µg per mouse, or ²²⁵Ac-DARA at 400 nCi and 200 nCi per 0.3 µg of antibody. Mice were observed for tumor progression for 40 days and any mouse whose tumor reached 4,000 mm³ volume, or became necrotic, was humanely euthanized **(****FIGS. 9A** and **9B**).

To evaluate the side effects of RIT, we performed a comprehensive safety evaluation of ²²⁵Ac-DARA by monitoring the weight of the mice (**FIG. 10A**) and assessing hematologic parameters and systemic toxicity (kidneys and liver; **FIG. 10B**). The weight of mice in the 200 nCi ²²⁵Ac-DARA group did not change throughout the experiment while mice in the 400 nCi group demonstrated only transient weight loss during the 2nd week post treatment, with rapid recovery noted in the 3rd week post-treatment. The evaluation of hematologic and toxicity parameters demonstrated that there was no statistically significant difference in any of the ²²⁵Ac-labeled versus unlabeled groups in any parameter tested. This indicated that there was no impact of the presence of ²²⁵Ac conjugate on blood parameters or evidence of increased liver damage as measured by aspartate aminotransferase (AST) and alanine transaminase (ALT) levels in treated mice. Further, there was no change in creatinine and blood urea nitrogen (BUN) demonstrating the absence of any apparent kidney toxicity.

The following aspects are disclosed in this application:
Aspect 1. A method of treating a subject having a proliferative disorder, the method comprising: administering to the subject an effective amount of an anti-CD33 antibody and an effective amount of an anti-CD38 antibody.
Aspect 2. The method of aspect 1, wherein the anti-CD33 antibody comprises HuM195.
Aspect 3. The method of aspects 1 or 2, wherein the anti-CD38 antibody comprises daratumumab, Mor202, or SAR650984; such as daratumumab.
Aspect 4. The method of according to any one of aspects 1 to 3, wherein one or both of the anti-CD33 antibody and the anti-CD38 antibody are labelled with a radioisotope.
Aspect 5. The method of according to any one of aspects 1 to 4, wherein the anti-CD33 antibody and the anti-CD38 antibody each independently comprise ¹³¹I, ¹²⁵I, ¹²³I, ⁹⁰Y, ¹⁷⁷Lu, ¹⁸⁶Re, ¹⁸⁸Re, ⁸⁹Sr, ¹⁵³Sm, ³²P, ²²⁵Ac, ²¹³Bi, ²¹³Po, ²¹¹At, ²¹²Bi, ²¹³Bi, ²²³Ra, ²²⁷Th, ¹⁴⁹Tb, ¹³⁷Cs, ²¹²Pb or ¹⁰³Pd.
Aspect 6. The method of according to any one of aspects 1 to 5, wherein the anti-CD33 antibody and the anti-CD38 antibody are each independently labelled with ¹³¹I or ²²⁵Ac.
Aspect 7. The method of according to any one of aspects 1 to 6, wherein the anti-CD33 antibody is ²²⁵Ac-labelled and the effective amount of the ²²⁵Ac-labelled anti-CD33 antibody comprises a dose of 0.5 to 4 uCi/kg subject body weight.
Aspect 8. The method of according to any one of aspects 1 to 7, wherein the anti-CD38 antibody is ²²⁵Ac-labelled and the effective amount of the ²²⁵Ac-labelled anti-CD38 antibody comprises a dose of 0.5 to 4 uCi/kg subject body weight.
Aspect 9. The method of according to any one of aspects 1 to 8, wherein the effective amount of the anti-CD33 antibody and the anti-CD38 antibody each independently comprise a dose of less than 16mg/kg of subject body weight.
Aspect 10. The method of according to any one of aspects 1 to 9, wherein the anti-CD33 antibody and the anti-CD38 antibody are administered at the same time, or wherein the anti-CD33 antibody and the anti-CD38 antibody are administered sequentially.
Aspect 11. The method of according to any one of aspects 1 to 10, wherein the anti-CD33 antibody is administered before the anti-CD38 antibody, or the anti-CD33 antibody is administered after the anti-CD38 antibody, or the anti-CD33 antibody is administered both before and after the anti-CD38 antibody, or the anti-CD38 antibody is administered both before and after the anti-CD33 antibody.
Aspect 12. The method of according to any one of aspects 1 to 11, wherein the anti-CD33 antibody and the anti-CD38 antibody are each independently administered according to a dosing schedule selected from the group consisting of once every week, once every two weeks, once every three weeks, once every four weeks, once every five weeks, once every six weeks, once every seven weeks, and once every eight weeks throughout a treatment period, wherein the treatment period includes at least two doses.
Aspect 13. The method of according to any one of aspects 1 to 12, wherein the anti-CD33 antibody and the anti-CD38 antibody are each independently administered via two injections separated by 5, 6, 7, 8, 9, or 10 days.
Aspect 14. The method of according to any one of aspects 1 to 13, wherein the proliferative disease is a hematological disease or a solid tumor.
Aspect 15. The method of according to any one of aspects 1 to 14, wherein the proliferative disease is multiple myeloma, relapsed or refractory multiple myeloma, acute myeloid leukemia, chronic myeloid leukemia, chronic lymphocytic leukemia, acute lymphoblastic leukemia, non-Hodgkin lymphoma, or Hodgkin lymphoma.
Aspect 16. The method of according to any one of aspects 1 to 15, further comprising: administering one or more therapeutic agents.
Aspect 17. The method of aspect 16, wherein the one or more therapeutic agents comprise an anti-myeloma agent, a chemotherapeutic agent, an anti-inflammatory agent, an immunosuppressive, an immunomodulatory agent, a cytokine, a hormone, or a combination thereof.
Aspect 18. The method of aspect 16, wherein the one or more therapeutic agents comprise an antimyeloma agent, wherein the antimyeloma agent is selected from dexamethasone, melphalan, doxorubicin, bortezomib, lenalidomide, prednisone, carmustine, etoposide, cisplatin, vincristine, cyclophosphamide, and thalidomide.
Aspect 19. The method of aspect 16, wherein, wherein the one or more therapeutic agents comprise granulocyte colony-stimulating factor.
Aspect 20. A method of treating a subject having relapsed or refractory multiple myeloma, the method comprising: administering to the subject ²²⁵Ac-HuM195 in a dose of 0.5 to 4 uCi/kg subject body weight and ²²⁵Ac-daratumumab in a dose of 0.5 to 4 uCi/kg subject body weight.

## Claims

1. An anti-CD33 antibody and an anti-CD38 antibody for use in treating a subject having a proliferative disorder, comprising:
an effective amount of the anti-CD33 antibody and the anti-CD38 antibody,
wherein the anti-CD33 antibody and the anti-CD38 antibody each independently comprise a label selected from ²²⁵Ac;
wherein the proliferative disorder is acute myeloid leukemia or multiple myeloma;
wherein the anti-CD33 antibody and anti-CD38 antibody are attached to the label by a conjugated chelating agent;
wherein the chelating agent attached to the anti-CD38 antibody is 1,4,7,10-tetraazacyclododecance-1,4,7,10-tetraacetic acid (DOTA), and
wherein the anti-CD33 antibody is lintuzumab and the anti-CD38 antibody is daratumumab.

2. An anti-CD33 antibody and an anti-CD38 antibody for use according to claim 1, wherein the effective amount of the ²²⁵Ac-labelled antibody comprises a dose of 0.5 to 4 µCi/kg subject body weight.

3. An anti-CD33 antibody and an anti-CD38 antibody for use according to claim 2, wherein either or both of the anti-CD33 antibody and the anti-CD38 antibody comprise a labeled fraction and an unlabeled fraction in a ratio of 0.1:1 to 1:1 labeled to unlabeled.

4. An anti-CD33 antibody and an anti-CD38 antibody for use according to claim 1, wherein the effective amount of the anti-CD33 antibody and the anti-CD38 antibody each independently comprise a dose of less than 16mg/kg of subject body weight.

5. An anti-CD33 antibody and an anti-CD38 antibody for use according to claim 1, wherein the anti-CD33 antibody and the anti-CD38 antibody are administered at the same time, or wherein the anti-CD33 antibody and the anti-CD38 antibody are administered sequentially; and wherein the anti-CD33 antibody and the anti-CD38 antibody are each independently administered according to a dosing schedule selected from the group consisting of once every week, once every two weeks, once every three weeks, once every four weeks, once every five weeks, once every six weeks, once every seven weeks, and once every eight weeks throughout a treatment period, wherein the treatment period includes at least two doses.

6. An anti-CD33 antibody and an anti-CD38 antibody for use according to claim 1, wherein the anti-CD33 antibody and the anti-CD38 antibody are administered at the same time, or wherein the anti-CD33 antibody and the anti-CD38 antibody are administered sequentially; and wherein the anti-CD33 antibody and the anti-CD38 antibody are each independently administered via two injections separated by 5, 6, 7, 8, 9, or 10 days.

7. An anti-CD33 antibody and an anti-CD38 antibody for use according to claim 1, wherein the proliferative disease is acute myeloid leukemia.

8. An anti-CD33 antibody and an anti-CD38 antibody for use according to claim 1, wherein the proliferative disease is multiple myeloma.

9. An anti-CD33 antibody and an anti-CD38 antibody for use according to claim 11, wherein the multiple myeloma is relapsed or refractory multiple myeloma.

10. An anti-CD33 antibody and an anti-CD38 antibody for use according to claim 8 or 9, further comprising one or more antimyeloma agents selected from: dexamethasone, melphalan, doxorubicin, bortezomib, lenalidomide, prednisone, carmustine, etoposide, cisplatin, vincristine, cyclophosphamide, and thalidomide.

11. An anti-CD33 antibody and an anti-CD38 antibody for use according to claim 1, further comprising granulocyte colony-stimulating factor.

## Patentansprüche

1. Anti-CD33-Antikörper und Anti-CD38-Antikörper zur Verwendung bei der Behandlung eines Patienten, der eine proliferative Erkrankung aufweist, umfassend:
eine wirksame Menge des Anti-CD33-Antikörpers und des Anti-CD38-Antikörpers,
wobei der Anti-CD33-Antikörper und der Anti-CD38-Antikörper jeweils unabhängig voneinander eine Markierung umfassen, ausgewählt aus ²²⁵Ac;
wobei die proliferative Erkrankung akute myeloische Leukämie oder Multiples Myelom ist;
wobei der Anti-CD33-Antikörper und der Anti-CD38-Antikörper durch einen konjugierten Chelatbildner an die Markierung gebunden sind;
wobei der an den Anti-CD38-Antikörper gebundene Chelatbildner 1,4,7,10-Tetraazacyclododecan-1,4,7,10-tetraessigsäure (DOTA) ist, und
wobei der Anti-CD33-Antikörper Lintuzumab ist und der Anti-CD38-Antikörper Daratumumab ist.

2. Anti-CD33-Antikörper und Anti-CD38-Antikörper zur Verwendung nach Anspruch 1, wobei die wirksame Menge des ²²⁵Ac-markierten Antikörpers eine Dosis von 0,5 bis 4 µCi/kg Körpergewicht des Patienten umfasst.

3. Anti-CD33-Antikörper und Anti-CD38-Antikörper zur Verwendung nach Anspruch 2, wobei einer oder beide des Anti-CD33-Antikörpers und des Anti-CD38-Antikörpers eine markierte Fraktion und eine unmarkierte Fraktion in einem Verhältnis von 0,1:1 bis 1:1 markiert zu unmarkiert umfassen.

4. Anti-CD33-Antikörper und Anti-CD38-Antikörper zur Verwendung nach Anspruch 1, wobei die wirksame Menge des Anti-CD33-Antikörpers und des Anti-CD38-Antikörpers jeweils unabhängig voneinander eine Dosis von weniger als 16 mg/kg Körpergewicht des Patienten umfasst.

5. Anti-CD33-Antikörper und Anti-CD38-Antikörper zur Verwendung nach Anspruch 1, wobei der Anti-CD33-Antikörper und der Anti-CD38-Antikörper gleichzeitig verabreicht werden oder wobei der Anti-CD33-Antikörper und der Anti-CD38-Antikörper nacheinander verabreicht werden; und wobei der Anti-CD33-Antikörper und der Anti-CD38-Antikörper jeweils unabhängig voneinander gemäß einem Dosierungsschema verabreicht werden, ausgewählt aus der Gruppe bestehend aus einmal pro Woche, einmal alle zwei Wochen, einmal alle drei Wochen, einmal alle vier Wochen, einmal alle fünf Wochen, einmal alle sechs Wochen, einmal alle sieben Wochen und einmal alle acht Wochen über einen Behandlungszeitraum, wobei der Behandlungszeitraum mindestens zwei Dosen beinhaltet.

6. Anti-CD33-Antikörper und Anti-CD38-Antikörper zur Verwendung nach Anspruch 1, wobei der Anti-CD33-Antikörper und der Anti-CD38-Antikörper gleichzeitig verabreicht werden oder wobei der Anti-CD33-Antikörper und der Anti-CD38-Antikörper nacheinander verabreicht werden; und wobei der Anti-CD33-Antikörper und der Anti-CD38-Antikörper jeweils unabhängig voneinander über zwei Injektionen im Abstand von 5, 6, 7, 8, 9 oder 10 Tagen verabreicht werden.

7. Anti-CD33-Antikörper und Anti-CD38-Antikörper zur Verwendung nach Anspruch 1, wobei die proliferative Erkrankung akute myeloische Leukämie ist.

8. Anti-CD33-Antikörper und Anti-CD38-Antikörper zur Verwendung nach Anspruch 1, wobei die proliferative Erkrankung Multiples Myelom ist.

9. Anti-CD33-Antikörper und Anti-CD38-Antikörper zur Verwendung nach Anspruch 11, wobei das Multiple Myelom rezidiviertes oder refraktäres Multiples Myelom ist.

10. Anti-CD33-Antikörper und Anti-CD38-Antikörper zur Verwendung nach Anspruch 8 oder 9, der weiter ein oder mehrere Anti-Myelom-Mittel umfasst, ausgewählt aus: Dexamethason, Melphalan, Doxorubicin, Bortezomib, Lenalidomid, Prednison, Carmustin, Etoposid, Cisplatin, Vincristin, Cyclophosphamid und Thalidomid.

11. Anti-CD33-Antikörper und Anti-CD38-Antikörper zur Verwendung nach Anspruch 1, der weiter Granulozytenkolonie-stimulierenden Faktor umfasst.

## Revendications

1. Anticorps anti-CD33 et anticorps anti-CD38 destinés à être utilisés dans le traitement d'un sujet présentant un trouble prolifératif, comprenant :
une quantité efficace de l'anticorps anti-CD33 et de l'anticorps anti-CD38,
dans lesquels l'anticorps anti-CD33 et l'anticorps anti-CD38 comprennent chacun indépendamment un marqueur choisi parmi ²²⁵Ac ;
dans lesquels le trouble prolifératif est la leucémie myéloïde aiguë ou le myélome multiple ;
dans lesquels l'anticorps anti-CD33 et l'anticorps anti-CD38 sont attachés au marqueur par un agent chélateur conjugué ;
dans lesquels l'agent chélateur attaché à l'anticorps anti-CD38 est l'acide 1,4,7,10-tétraazacyclododécance-1,4,7,10-tétraacétique (DOTA), et
dans lesquels l'anticorps anti-CD33 est le lintuzumab et l'anticorps anti-CD38 est le daratumumab.

2. Anticorps anti-CD33 et anticorps anti-CD38 destinés à être utilisés selon la revendication 1, dans lesquels la quantité efficace de l'anticorps marqué au ²²⁵Ac comprend une dose de 0,5 à 4 µCi/kg de poids corporel du sujet.

3. Anticorps anti-CD33 et anticorps anti-CD38 destinés à être utilisés selon la revendication 2, dans lesquels un ou les deux parmi l'anticorps anti-CD33 et l'anticorps anti-CD38 comprennent une fraction marquée et une fraction non marquée en un rapport de 0,1:1 à 1:1 de marquée à non marquée.

4. Anticorps anti-CD33 et anticorps anti-CD38 destinés à être utilisés selon la revendication 1, dans lesquels la quantité efficace de l'anticorps anti-CD33 et la quantité efficace de l'anticorps anti-CD38 comprennent chacune indépendamment une dose inférieure à 16 mg/kg de poids corporel du sujet.

5. Anticorps anti-CD33 et anticorps anti-CD38 destinés à être utilisés selon la revendication 1, dans lesquels l'anticorps anti-CD33 et l'anticorps anti-CD38 sont administrés en même temps, ou dans lesquels l'anticorps anti-CD33 et l'anticorps anti-CD38 sont administrés séquentiellement ; et dans lesquels l'anticorps anti-CD33 et l'anticorps anti-CD38 sont administrés chacun indépendamment selon un schéma posologique choisi dans le groupe constitué d'une fois par semaine, une fois toutes les deux semaines, une fois toutes les trois semaines, une fois toutes les quatre semaines, une fois toutes les cinq semaines, une fois toutes les six semaines, une fois toutes les sept semaines et une fois toutes les huit semaines pendant une période de traitement, dans lesquels la période de traitement inclut au moins deux doses.

6. Anticorps anti-CD33 et anticorps anti-CD38 destinés à être utilisés selon la revendication 1, dans lesquels l'anticorps anti-CD33 et l'anticorps anti-CD38 sont administrés en même temps, ou dans lesquels l'anticorps anti-CD33 et l'anticorps anti-CD38 sont administrés séquentiellement ; et dans lesquels l'anticorps anti-CD33 et l'anticorps anti-CD38 sont administrés chacun indépendamment via deux injections séparées de 5, 6, 7, 8, 9 ou 10 jours.

7. Anticorps anti-CD33 et anticorps anti-CD38 destinés à être utilisés selon la revendication 1, dans lesquels la maladie proliférative est la leucémie myéloïde aiguë.

8. Anticorps anti-CD33 et anticorps anti-CD38 destinés à être utilisés selon la revendication 1, dans lesquels la maladie proliférative est le myélome multiple.

9. Anticorps anti-CD33 et anticorps anti-CD38 destinés à être utilisés selon la revendication 11, dans lesquels le myélome multiple est un myélome multiple récidivant ou réfractaire.

10. Anticorps anti-CD33 et anticorps anti-CD38 destinés à être utilisés selon la revendication 8 ou 9, comprenant en outre un ou plusieurs agents antimyélomes choisis parmi : la dexaméthasone, le melphalan, la doxorubicine, le bortézomib, le lénalidomide, la prednisone, la carmustine, l'étoposide, le cisplatine, la vincristine, le cyclophosphamide et le thalidomide.

11. Anticorps anti-CD33 et anticorps anti-CD38 destinés à être utilisés selon la revendication 1, comprenant en outre un facteur de stimulation des colonies de granulocytes.
